# EUROPÄISCHE PATENTSCHRIFT

(11) **EP 3 995 135 B1**
(45) Veröffentlichungstag und Bekanntmachung des Hinweises auf die Patenterteilung: **13.07.2022**
(21) Anmeldenummer: 20206800.3
(22) Anmeldetag: 10.11.2020
(51) Int. Cl.: A61K 31/137, A61K 9/20

(54) **DARREICHUNGSFORMEN MIT VERLÄNGERTER FREISETZUNG EINES SALZES VON TAPENTADOL MIT L-(+)-WEINSÄURE**
PROLONGED RELEASE DOSAGE FORM OF TAPENTADOL L-(+)- TARTARIC ACID SALT
FORMES PHARMACEUTIQUES AVEC LIBÉRATION PROLONGÉE D'UN SEL DE TAPENTADOL AVEC L - (+) - ACIDE TARTRIQUE

(43) Veröffentlichungstag der Anmeldung: 11.05.2022
(73) Patentinhaber: Grünenthal GmbH, 52078 Aachen (DE)
(72) Erfinder: BERTRAM, Ulrike, 52066 Aachen (DE); REINHOLD, Ulrich, 52076 Aachen (DE); GROSSE, Christian, 52072 Aachen (DE); HARTMANN, Carmen, 50859 Köln (DE)
(74) Vertreter: Kutzenberger Wolff & Partner

(56) Entgegenhaltungen:
- EP-A1- 2 942 054
- WO-A1-2009/092601
- WO-A1-2017/085734
- US-A1- 2015 283 087

## Beschreibung

Die Erfindung betrifft eine Tablette, die eine verlängerte Freisetzung von Tapentadol bereitstellt, wobei Tapentadol in Form eines Salzes mit Weinsäure, insbesondere L-(+)-Weinsäure, vorliegt. Die Tablette hat zufriedenstellende mechanische Eigenschaften, z. B. im Hinblick auf Bruchfestigkeit und Friabilität, und lässt sich unter stark erleichterten Tablettierbedingungen, insbesondere bei verminderter Presskraft, herstellen.

Tabletten werden hergestellt, indem man eine Presskraft auf ein Pulver einwirken lässt und das Pulver in der Prägeform einer Presse zu einer kompaktierten Form konsolidiert. Bei der Herstellung einer Tablette finden zwei Prozesse gleichzeitig statt: Kompaktierung und Kompression. Definitionsgemäß ist die Kompaktierung eine Erhöhung der mechanischen Stärke des Pulvers unter Krafteinwirkung aufgrund der Konsolidierung der Partikel. Die Kompaktierung steht somit mit Partikelkonsolidierung und -bindung in Zusammenhang, was eine direkte Auswirkung auf die Bruchfestigkeit der Tablette hat. Kompression ist andererseits als eine Verminderung des Schüttvolumens des Pulvers unter Krafteinwirkung durch Verdrängung von Luft zwischen den Partikeln definiert. Die Kompression führt zu einer Verminderung des Leerraums zwischen festen Partikeln, was eine Abnahme der Porosität einer Tablette bedeutet (Genaueres findet sich z. B. bei Y. Qiu et al., Developing Solid Oral Dosage Forms, Pharmaceutical Theory & Practice, 2. Aufl., Elsevier, 2017, S. 940-942).

Die Tablettenherstellung wird durch eine Reihe voneinander abhängiger Parameter beeinflusst, einschließlich
- den gewünschten Eigenschaften der Tablette (z. B. mechanische Stärke, Gewichtsvariabilität, Zerfallszeit, Größe und Form),
- der Beschaffenheit und der Menge der Exzipienten des zu Tabletten zu formenden festen Materials und
- den Bedingungen in der Tablettiermaschine (z. B. Presskraft, Tablettiergeschwindigkeit, Verweilzeit des Stempels in der Prägeform, Stempelgeometrie, Werkzeugmaterial usw.).

Was die gewünschten Eigenschaften der Tablette betrifft, so hängen diese in hohem Maße von pharmakologischen, d. h. pharmakokinetischen und pharmakodynamischen, Betrachtungen ab. Die in einer Tablette zu enthaltende Arzneimitteldosis wird durch die Wirksamkeit des Arzneimittels und die vorgesehene Verabreichungshäufigkeit bestimmt. Die Freisetzungscharakteristika bestimmen die Beschaffenheit und Menge an Exzipienten sowie die Gesamtverteilung von Exzipienten in der Tablette. Eine Matrixretardierung beispielsweise erfordert beträchtliche Mengen an Matrixmaterial mit verlängerter Freisetzung, in dem das Arzneimittel eingebettet ist. Eine ausreichende mechanische Stärke ist erforderlich, um z. B. das Ausstoßen der kompaktierten Formen aus der Prägeform beim Tablettierverfahren und die Freisetzung von Tabletten aus einer geeigneten Verpackung wie einer Durchdrückpackung zu ermöglichen.

Was die Beschaffenheit und Menge an Exzipienten betrifft, so werden Pulverexzipienten als Bindemittel für die Kohäsion, als Füllstoffe für die Dehnfestigkeit der Tablette, als Schmiermittel für einen erfolgreiches Verarbeiten einer Mischung oder eines Granulats zu einer Tablette, als Sprengmittel zur Erleichterung des Zerfalls der Tablette in vivo, als Materialien für eine kontrollierte Freisetzung für ein retardiertes Auflösen und manchmal als Gleitmittel für Mischungen mit schlechter Fließfähigkeit. verwendet. Die Exzipienten sind jeweils vorwiegend entweder elastisch oder plastisch oder spröde. Elastische Partikel verformen sich während der Kompression bis zu ihrem elastischen Limit und brechen dann. Vor dem Erreichen dieser Dehngrenze ist die Verformung des elastischen Materials reversibel. Nach Entfernen der Kompressionskräfte expandieren elastische Pulver (Relaxation). Bei der plastischen Deformation bleibt die Form nach Entfernen der Kompressionskräfte erhalten and sie deformiert daher ohne Wiederherstellung des Ausgangszustands, unter Bereitstellung einer guten Teilchenbindung. Eine spröde Komponente wird unter Kompressionsbelastung brechen, wenn die Kraft die Deformationsgrenze übersteigt. Während sich nach einem spröden Bruch eine größere Oberfläche bildet, führt dieser auch zu einer größeren Reibung und einem größeren Widerstand gegenüber Bewegung in der Prägeform während der Kompression. Plastische und spröde Exzipienten bilden physikalische Formen und verleihen der Form nach der Kompression Stabilität.

Was die Bedingungen in der Tablettiermaschine betrifft, so kommen bei modernen hochentwickelten Tablettiermaschinen jeweils die gleichen allgemeinen Betriebskonzepte zur Anwendung: Füllen der Prägeform, Pressen der Tablette, Ausstoß der Tablette und Abschaben des Tisches. Die Qualität der kompaktierten Form hängt von der Kompression und Konsolidierung der Pulvermasse, der Dekompression der kompaktierten Form, dem Ausstoßen aus der Prägeform und dem anschließenden Abschaben vom unteren Stempel ab. Da diese viskoelastischen Eigenschaften zeitabhängig sind, wird die Tablettenqualität sowohl durch die Größe als auch die Rate der Anwendung (und das Lösen) der Presskraft beeinflusst.

Tablettierstempel werden zum Verpressen einer Mischung oder eines Granulats zu einer kompaktierten Einheitsdosis verwendet.

Bei Tablettiermaschinen für die Walzenkompaktierung (Rotationstablettenpressen) wird zunächst der untere Stempel abgesenkt, so dass die Zuführungsvorrichtung Pulver in eine Prägeform abgeben kann. Mit einem Schaber wird überschüssiges Pulver entfernt, so dass die Formulierung eben mit dem oberen Rand der Prägeform ist. Die Menge an Pulver in der Prägeform ist die Füllmenge. Dann wird der obere Stempel in die Prägeform abgesenkt. Die Vorkompressionswalzen üben Druck aus, durch den die Füllmenge in der Prägeform von Luft befreit wird, indem die Teilchen enger zusammen gezwängt werden. Hierauf hin üben die Hauptkompressionswalzen Kraft aus, durch die das Pulver zu einer kompaktierten Form verpresst wird. Der obere Stempel wird dann durch die obere Hebenocke aus der Prägeform herausgezogen. Anschließend wird der untere Stempel von der Ausstoßnocke nach oben geschoben, wodurch die kompaktierte Form aus der Prägeform ausgestoßen wird, so dass sie ein Schaber entfernen kann. Die Kompression ermöglicht eine Bindung von Teilchen an Teilchen zum Zweck der Herstellung einer Tablette mit einer zur Aufrechterhaltung ihrer Form und ihres Aussehens ausreichenden Kohäsionsstärke bzw. Härte.

Bei Tablettiermaschinen mit Exzenterpresse (Exzentertablettenpressen) gibt es keine Vorkompression und die Kraft wird nicht über Kompressionswalzen übertragen. Das Gesamtprinzip von Kompression und Kompaktierung ist jedoch sehr ähnlich.

Gibt man Pulver in eine Prägeform, so befindet sich zwischen den Partikeln Leerraum. Es wurde gezeigt, dass sich die Partikel bei der Kompression des Pulvers konsolidieren und die Pulverfüllmenge von Luft befreit wird. Durch die Prägeform wird die Bewegung des Pulvers behindert und Zeit für die kohäsive Bindung von Partikeln aneinander bereitgestellt. Hierauf folgen eine Umlagerung der Partikel, plastische und elastische Deformation und anschließend die Fragmentierung der spröden Komponenten, nachdem sie ihre Deformationsgrenze erreicht haben. Der Druck ermöglicht die Ausbildung von Bindungen zwischen den Partikeln, die der kompaktierten Form Dehnfestigkeit verleihen. Nach dem Ausstoßen aus der Prägeform durchläuft die kompaktierte Form eine Art elastischer Entspannung (Relaxation), bei der die elastischen Materialien zum Teil wieder ihre Formen vor der Kompression annehmen. Durch die Kohäsion der komprimierten Partikel bleibt die Form der kompaktierten Form nach der Kompression erhalten.

Einige der Deformationscharakteristika sind zeitabhängig und daher können Maschinencharakteristika einen großen Einfluss auf die Tablettierleistung haben. Diese Charakteristika bestimmen die Rate der Kraftanwendung, die Verweilzeit (d. h. die Zeit der maximalen Presskraft, die vom Durchmesser der Fläche des Stempelkopfes und der Tangentialgeschwindigkeit abhängt) und die Dekompressionsrate. Bei Materialien, die eine plastische Deformation durchmachen, steht bei erhöhter Maschinengeschwindigkeit typischerweise weniger Zeit für die Belastungsrelaxation zur Verfügung.

Tabletten müssen dazu fähig sein, den Belastungen von Herstellung, Verpackung, Versendung und Verteilung zu widerstehen. Gemäß den Quality Attribute Considerations for Chewable Tablets, Guidance for Industry der FDA vom August 2018 sollten Kautabletten eine Härte von < 12 kp (~19,6 N) aufweisen, wobei man sich bewusst ist, dass Tablettengröße und -form bei Festlegung einer annehmbaren Tablettenhärte von Bedeutung sind. Bei einer sehr kleinen Tablette beispielsweise könnte ein Härtewert von 20 N recht hoch sein. Weiterhin wurde empfohlen, dass schnell zerfallende Tabletten mit sofortiger Freisetzung des Arzneimittels eine Härte von etwa 70 bis 100 N aufweisen sollten, während Tabletten mit verlängerter Freisetzung des Arzneimittels eine Härte von etwa 100 bis 200 N aufweisen sollten. Je größer die Tabletten, desto härter müssen sie sein, um der Abnutzung während Herstellung, Verpackung, Versendung und Verteilung zu widerstehen. Im Hinblick auf Tabletten mit verlängerter Freisetzung sollte die Tablettenhärte deshalb mindestens 100 N, vorzugsweise mindestens 150 N, betragen.

Innerhalb gewisser Grenzen ist die Tablettenhärte eine Funktion der Presskraft; je höher die Presskraft, desto höher die Tablettenhärte. Bei einer gegebenen Exzipientenmischung lässt sich eine erhöhte Tablettenhärte somit häufig erzielen, indem man die Presskraft erhöht. Dies kann jedoch gleichzeitig eine Verminderung der Maschinengeschwindigkeit erfordern, um die Verweilzeit richtig einzustellen, wodurch die Tablettengesamtausbeute pro Stunde reduziert wird. Der Grund hierfür ist, dass sowohl die Bindung zwischen den Teilchen als auch die adhäsive Bindung nicht nur durch die Presskraft, sondern auch durch die Stempelverweilzeit, während der das Pulver in der Prägeform komprimiert wird, beeinflusst werden können. Die Verweilzeit bezieht sich auf die Zeitspanne, während der ein Stempel Pulver in der Prägeform komprimiert. Sie ist eine Funktion der Maschinengeschwindigkeit, wobei eine schnellere Tablettierung weniger Zeit zum Komprimieren des Pulvers in der Prägeform durch den Stempel gestattet. Bei einer Verlängerung der Verweilzeit steht mehr Zeit für die Partikeldeformation, für die Fragmentation zum Erhöhen der Oberfläche und zur Ausbildung von Bindungen zwischen Partikeln zur Verfügung. Verändert man die Presskraft, so sollte man außerdem beachten, dass hierdurch nicht nur die Tablettenhärte beeinflusst wird. Härtere Tabletten können längere Zerfallszeiten und Auflösungsraten haben. Bei der Suche nach einem Kompromiss zwischen Presskraft und Verweilzeit sind Tablettenattribute in Betracht zu ziehen (Genaueres findet sich z. B. bei M.T. Ende et al., Chemical Engineering In The Pharmaceutical Industry, Drug Product Design, Development, and Modeling, 2. Aufl., Wiley, 2019, S. 228-232; J. Swarbrick, M. Bogda, Encylopedia of Pharmaceutical Technology, 3. Aufl., Informa Healthcare, 2007, Tablet Compression: Machine Theory, Design, and Process Troubleshooting, 3611-2629).

Eine Erhöhung der Presskraft zur Verbesserung der Tablettenhärte kann somit eine Reduktion der Maschinengeschwindigkeit erfordern, was zu ökonomischen Nachteilen aufgrund eines reduzierten Outputs führt.

Weiterhin können die Kompressionskräfte aufgrund von Materialeigenschaften (werkzeugbedingte Einschränkungen) nicht *ad ultimo* erhöht werden. Die Tablettenwerkzeugstärke ist eine Funktion der Größe, der Form und des Füllgewichts der Tablette. Es gibt daher mehrere Tablettenformen und -größen, die maximale Kompressionskräfte von weniger als 10 kN gestatten. Infolgedessen werden Tablettiermaschinen, die mit Stempeln einer solchen Größe und Form ausgestattet sind, bereits bei Kompressionskräften nahe ihrer maximalen Werkzeugkraft betrieben, und unter diesen Bedingungen besteht wenig Manövrierraum für eine Erhöhung der Presskraft zum Erhöhen der Tablettenhärte (Genaueres findet sich z. B. bei L.L. Augsburger et al., Pharmaceutical Dosage Forms: Tablets, 3. Aufl., informa healthcare, 2008, S. 26-30; J. Zheng, Formulation and Analytical Development for Low-Dose Oral Drug Products, Wiley 2009, 150-155).

Aus dem Stand der Technik sind verschiedene Tapentadol enthaltende Tabletten bekannt.

Die WO 2003/035054 A1 betrifft eine pharmazeutische Formulierung, die durch eine verzögerte Freisetzung von Tapentadol HCl in einer Matrix mit verzögerter Freisetzung des Wirkstoffs gekennzeichnet ist. Diese Matrix enthält zwischen 1 und 80 Gew.-% mindestens eines hydrophilen oder hydrophoben Polymers als eine pharmazeutisch unbedenkliche Matrix bildendem Mittel. Das Granulat wurde auf einer EKO-Exzenterpresse (Korsch) zu länglichen Tabletten mit einer Größe von 6 × 15 mm und Bruchrille verpresst.

Die EP 2 942 054 A1 betrifft eine pharmazeutische Formulierung mit langsamer Freisetzung, die Tapentadol HCl in einer Matrix mit langsamer Freisetzung enthält, wobei die Matrix zwischen 15 und 50 Gew.-% Mono-, Di- und Triglyceride gesättigter Fettsäuren mit einer Kettenlänge zwischen 16 und 22 Kohlenstoffatomen oder eine Mischung davon enthält. Der Einfluss von Presskraft und Tablettendimensionen auf die Tablettenhärte und das Auflösungsverhalten wurde untersucht. Bei einer konstanten Kraft von 27 kN konnte eine Tablettenhärte von lediglich 53 bis 79 N erzielt werden, während niedrigere Kräfte eine noch schlechtere Tablettenhärte lieferten.

Die WO 2008/051617 A2 betrifft ein Verfahren zur Herstellung einer Trockengranulatzusammensetzung, bei dem man eine pharmazeutische Zusammensetzung unter Bildung eines oder mehrerer Rohlinge zu einer Härte von 800 bis 900 kPa verpresst und den einen oder die mehreren Rohlinge unter Bildung eines Granulats mit einem Schwinggranulator mahlt. Eine beispielhafte pharmazeutische Formulierung enthält Tapentadol HCl, Hypromellose, mikrokristalline Cellulose, kolloidales Siliciumdioxid und Magnesiumstearat. In den Figuren 7 bis 9 ist der Einfluss der Presskraft auf die Tablettenhärte gezeigt. Bei Kompressionskräften von 2000 bis 3000 lbs (~8,9 bis ~13,3 kN) wurden Tabletten mit einer Härte von etwa 7 bis 20 kp (~69 bis ~196 N) erhalten.

In der WO 2015/014980 A1 wird eine pharmazeutische Zusammensetzung offenbart, die (a) gelöstes Tapentadol HCl, (b) organisches Lösungsmittel mit einem Siedepunkt von 110° bis 350°C und (c) festen Träger umfasst. Verwendet man zur Herstellung der Tabletten eine Rotationspresse, so kann die HauptPresskraft im Bereich von 1 bis 50 kN, vorzugsweise 3 bis 40 kN, liegen. Die erhaltenen Tabletten können eine Härte von 30 bis 400 N, besonders bevorzugt von 50 bis 250 N, speziell bevorzugt von 30 bis 180 N und noch weiter bevorzugt von 40 bis 150 N aufweisen. Die WO 2009/092601 A1 offenbart Tapentadol oder die entsprechenden Salze in kontrolliert freisetzenden Matrix-Darreichungsformen, wie z.B. Tapentadol HCl Tabletten mit PEO und HPMC und einer Bruchfestigkeit von > 100N und einer Freisetzung über 600 Minuten.

D.V. Reddy et al., Journal of Pharmacy Research 2014. 8(10), 1368-1374 beschreiben die Herstellung von Tapentadol HCl-Tabletten mit retardierter Freisetzung unter Verwendung von durch Nassgranulation hergestellten Kombinationen von hydrophilen und hydrophoben Polymeren.

S.M. Afzal et al., Int J A PS BMS 2017, 6(1), 1-21 betrifft die Herstellung von Tapentadol HCl-Tabletten mit einer Matrix mit anhaltender Freisetzung durch ein Sinterverfahren.

S.K. Paramasivan et al., GSC Biological and Pharmaceutical Sciences, 2018, 04(03), 042-048 betrifft Tapentadol umfassende Tabletten, die durch direktes Verpressen in einer Rotationstablettenpresse hergestellt werden.

Die bekannten tapentadolhaltigen Tabletten sind nicht in jeder Hinsicht zufriedenstellend, insbesondere da die Kompression von Tabletten aus den Ausgangsmaterialien beträchtliche Kompressionskräfte erfordert, um eine zufriedenstellende mechanische Stärke, z. B. Härte, zu erzielen.

Es wäre wünschenswert, orale Darreichungsformen von Tapentadol bereitzustellen, die leicht herzustellen sind und die z. B. keine thermische Formgebung oder keine mehrfachen Beschichtungsschritte erfordern. Die oralen Darreichungsformen sollten vorzugsweise in Form von Tabletten, bevorzugt monolithischen Tabletten, bereitgestellt werden, d. h. die Herstellung einer Vielzahl von Partikeln sollte nicht erforderlich sein. Die Herstellung der oralen Darreichungsformen sollte auf Standardgerät in großindustriellem Maßstab mit hohem Durchsatz möglich sein, vorzugsweise durch Verpressen von Pulvermischungen, möglicherweise mit Granulation (Trockengranulation oder Nassgranulation), bevorzugt jedoch durch direktes Verpressen von Pulvermischungen.

Es besteht ein Bedarf an Formulierungen von Tapentadol, die sich mittels herkömmlicher Tablettiermaschinen bei hohen Maschinengeschwindigkeiten zu Tabletten verpressen lassen, die zufriedenstellende Tabletteneigenschaften bereitstellen, insbesondere im Hinblick auf Härte, und die eine breite Variation von Tablettengrößen und -formen erlauben, ohne dass die Werkzeugstärkengrenzen von herkömmlichem Tablettiergerät erreicht werden.

Aufgabe der Erfindung ist die Bereitstellung pharmazeutischer Darreichungsformen von Tapentadol, die im Vergleich zu den pharmazeutischen Darreichungsformen aus dem Stand der Technik Vorteile bieten.

Diese Aufgabe wurde durch den Gegenstand der Patentansprüche gelöst.

### KURZE DARSTELLUNG DER ERFINDUNG

Die Erfindung stellt eine Tapentadol umfassende pharmazeutische Darreichungsform für die zweimal tägliche orale Verabreichung bereit; wobei Tapentadol als ein Salz mit Weinsäure, insbesondere L-(+)-Weinsäure vorliegt; wobei die Darreichungsform eine verlängerte Freisetzung von Tapentadol bereitstellt; und wobei die in der pharmazeutischen Darreichungsform enthaltene gewichtsäquivalente Dosis von Tapentadol im Bereich von 10 bis 300 mg liegt, bezogen auf die freie Base von Tapentadol (Mᵣ = 221,3 g/mol).

Es wurde überraschenderweise gefunden, dass Salze von Tapentadol mit Weinsäure, insbesondere L-(+)-Weinsäure, von besonderem Nutzen für pharmazeutische Darreichungsformen sind, die eine verlängerte Freisetzung von Tapentadol bereitstellen. Es wurde überraschenderweise gefunden, dass im Vergleich zu herkömmlichem Tapentadolhydrochlorid die Herstellung von Salze von Tapentadol mit Weinsäure, insbesondere L-(+)-Weinsäure, umfassenden komprimierten Tabletten signifikant reduzierte Kompressionskräfte erfordert, um eine gewünschte Zielbruchfestigkeit der Tabletten zu erreichen. Dieser Effekt hängt vorteilhafterweise weder von der Teilchengröße der Salze von Tapentadol mit Weinsäure noch von der polymorphen Form des Salzes von Tapentadol mit Weinsäure ab. Insbesondere wird dieser Effekt auch bei verschiedenen anderen Exzipienten beobachtet, d. h. er kann den Eigenschaften des Salzes von Tapentadol mit Weinsäure, insbesondere L-(+)-Weinsäure, als solchem zugeschrieben werden.

Ein kristallines Produkt von Tapentadol mit Weinsäure ist aus der WO 2017/085734 A1 bekannt. Jedoch wird in die Reduktion der Kompressionskräfte zum Erreichen einer gewünschten Zielbruchfestigkeit der Tabletten in dieser Literaturstelle nicht angesprochen.

Darreichungsformen, die eine verlängerte Freisetzung von Tapentadol bereitstellen, sind ebenfalls bekannt, z. B. aus WO 03/035053 A1, WO 2006/002886 A1 und WO 2009/092601 A1. Jedoch wird in keiner dieser Literaturstellen die Reduktion der Kompressionskräfte zum Erreichen einer gewünschten Zielbruchfestigkeit der Tabletten angesprochen.

Gemäß der Gebrauchsanweisung enthalten die im Handel erhältlichen Tapentadol-Tabletten Palexia^{®} retard Tapentadol als Hydrochloridsalz, wobei der Tablettenkern zusätzlich Hypromellose, mikrokristalline Cellulose, feinteiliges Siliciumdioxid und Magnesiumstearat enthält. Palexia^{®} retard-Tabletten enthalten somit Hypromellose als Matrix mit verlängerter Freisetzung. Die Tablettenkerne sind mit einer Hypromellose, Lactose-monohydrat, Talkum, Macrogol 6000 und Farbstoffe umfassenden Zusammensetzung filmbeschichtet. Diese Tabletten entsprechen WO 03/035053 A1 und den hier im experimentellen Teil enthaltenen Vergleichsbeispielen.

### KURZE BESCHREIBUNG DER ZEICHNUNGEN

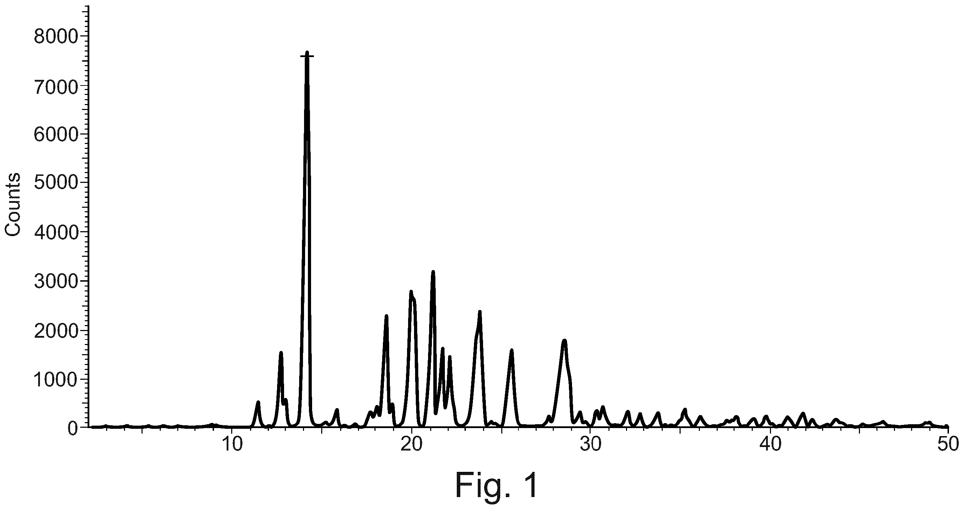

Figur 1 zeigt das XRPD-Diffraktogramm der gemäß WO 2017/085734 A1 erhaltenem kristallinen Form von Tapentadol und L-(+)-Weinsäure.

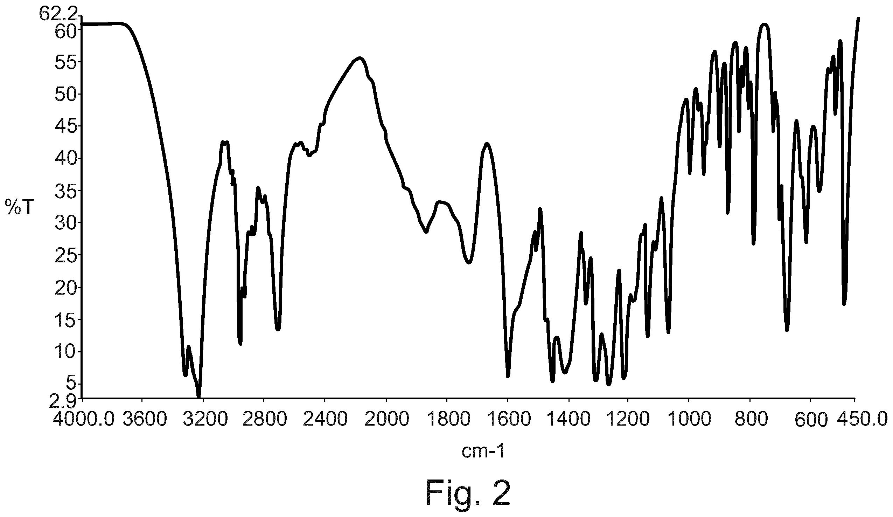

Figur 2 zeigt das Infrarot-Spektrum der gemäß WO 2017/085734 A1 erhaltenem kristallinen Form von Tapentadol und L-(+)-Weinsäure.
Figur 3 zeigt das XRPD-Diffraktogramm der kristallinen Form von Tapentadoltartrat gemäß Beispiel 1.

### AUSFÜHRLICHE BESCHREIBUNG DER ERFINDUNG

Ein erster Aspekt der Erfindung betrifft eine Tapentadol umfassende pharmazeutische Darreichungsform für die zweimal tägliche orale Verabreichung; wobei Tapentadol als ein Salz mit Weinsäure, insbesondere L-(+)-Weinsäure, vorliegt; wobei die Darreichungsform eine verlängerte Freisetzung von Tapentadol bereitstellt; und wobei die in der pharmazeutischen Darreichungsform enthaltene gewichtsäquivalente Dosis von Tapentadol im Bereich von 10 bis 300 mg liegt, bezogen auf die freie Base von Tapentadol.

Tapentadol, d. h. (-)-(1R,2R)-3-(3-Dimethylamino-1-ethyl-2-methylpropyl)phenol, ist ein synthetisches, zentral wirkendes Analgetikum, das bei der Behandlung von mäßigen bis schweren akuten oder chronischen Schmerzen wirkt. Die Synthese der freien Base von Tapentadol ist z. B. aus EP-A 693 475 bekannt.

Die erfindungsgemäße pharmazeutische Darreichungsform enthält Tapentadol als ein Salz mit Weinsäure, insbesondere L-(+)-Weinsäure. Es wird in Betracht gezogen, dass die erfindungsgemäße pharmazeutische Darreichungsform Mischungen verschiedener Salze von Tapentadol oder Mischungen von Salz(en) der freien Base von Tapentadol (z. B. die Nichtsalzform von Tapentadol)enthalten kann, jedoch liegt vorzugsweise die Gesamtmenge an in der pharmazeutischen Darreichungsform enthaltenem Tapentadol als ein Salz mit Weinsäure, insbesondere L-(+)-Weinsäure, vor.

Erfindungsgemäße Salze von Tapentadol mit Weinsäure schließen im Prinzip die Salze mit L-(+)-Weinsäure, D-(-)-Weinsäure, DL-(±)-Weinsäure und meso-Weinsäure ein. L-(+)-Weinsäure wird auch als (2R,3R)-Weinsäure bezeichnet, D-(-)-Weinsäure wird auch als (2S,3S)-Weinsäure bezeichnet und DL-(±)-Weinsäure als (2RS,3SR)-Weinsäure. Die entsprechenden Salze werden als L-(+)-Tartrate, D-(-)-Tartrate sowie DL-(±)-Tartrate bezeichnet.

Vorzugsweise handelt es sich bei dem Salz von Tapentadol mit Weinsäure um Salz von Tapentadol mit L-(+)-Weinsäure, das gegebenenfalls solvatisiert (z. B. hydratisiert) oder ansolvatisiert (z. B. anhydratisiert) sein kann. Das Salz von Tapentadol kann in Form einer einzelnen polymorphen Form oder als Mischung verschiedener polymorpher Formen in einem beliebigen Mischverhältnis vorliegen.

Gemäß einer bevorzugten Ausführungsform handelt es sich bei dem Salz von Tapentadol mit Weinsäure um das Salz mit L-(+)-Weinsäure, das in im Wesentlichen reiner kristalliner Form von Tapentadol-L-(+)-tartrat vorliegt.

Es wird auch in Betracht gezogen, dass in dem Salz von Tapentadol mit Weinsäure, insbesondere L-(+)-Weinsäure, diese beiden Komponenten im Verhältnis von 1:0.4 bis 1:2.1 vorliegen.

Salze mit einer im Wesentlichen 2:1 Stöchiometrie von Tapentadol zu Weinsäure, insbesondere L-(+)-Weinsäure, werden auch als Hemi-Salze bezeichnet und sind ebenfalls umfasst.

Gemäß einer bevorzugten Ausführungsform handelt es sich bei dem Salz von Tapentadol mit Weinsäure um ein kristallines L-(+)-Weinsäuresalz, d.h. um Tapentadol L-(+)-tartrat, mit charakteristischen Röntgenpulverbeugungspeaks bei 14,1, 20,0, 21,1 und 23.7 Grad 2Θ (± 0,2 Grad 2Θ); vorzugsweise gekennzeichnet durch einen oder mehrere weitere Röntgenpulverbeugungspeaks bei 12,7, 18,6, 21,6, 22,1 , 25,6 und/oder 28,5 Grad 2Θ (± 0,2 Grad 2Θ).

Alternativ kann das kristalline Tapentadol L-(+)-tartrat durch die folgenden Röntgenpulverbeugungspeaks bei Grad 2Θ ± 0,2 Grad 2Θ gekennzeichnet sein: 12,7, 14,1, 18,6, 20,0, 21,1, 21,6, 22,1, 23,7, 25,6 und/oder 28,5 gekennzeichnet sein.

Röntgenpulverbeugungsmessungen werden mit Cu K_{α} Strahlung bei Raumtemperatur durchgeführt. Ein geeignetes Gerät ist z.B. D8 ADVANCE Eco, Bruker. DSC Messungen werden gemäß ASTM D3418 durchgeführt. IR-Spektren werden auf einem FTIR-Spektrometer durchgeführt.

Ebenfalls alternativ kann das kristalline Tapentadol L-(+)-tartrat dadurch gekennzeichnet sein, dass in der Differential Scanning Kalorimetrie einen endothermen Übergang bei 132°C±3°C aufweist.

Weiterhin alternativ kann das kristalline Tapentadol L-(+)-tartrat charakterisiert sein durch Absorptionsbanden im Infrarot-Spektrum bei 3319, 3237, 2960, 1731, 1597, 1305, 1263, 1213, 791, 679 und 485 cm⁻¹.

Ein entsprechendes kristallines Tapentadol L-(+)-tartrat sowie Verfahren zu seiner Herstellung und Methoden der Charakterisierung sind aus der WO 2017/085734 A1 bekannt.

Für die Zwecke der Beschreibung soll ein Verweis auf "Tapentadol" das Salz von Tapentadol mit Weinsäure einschließen.

Das Salz von Tapentadol mit Weinsäure, insbesondere L-(+)-Weinsäure, kann in Form eines beliebigen Solvats, z. B. Hydrats, Ansolvats, z. B. Anhydrats, und einer beliebigen polymorphen Form, z. B. kristallinen Form und/oder amorphen Form, vorliegen.

Die Gewichtsäquivalentdosis von Tapentadol, die in der erfindungsgemäßen pharmazeutischen Darreichungsform enthalten ist, liegt im Bereich von 10 bis 300 mg, bezogen auf die freie Base von Tapentadol, z. B. 25 mg, 50 mg, 100 mg, 150 mg, 200 mg oder 250 mg. Die freie Base von Tapentadol (d. h. das freie Molekül) hat ein Molekulargewicht von 221,3 g/mol. 10 mg der freien Base von Tapentadol entsprechen somit 0,0452 mmol, während 300 mg der freien Base von Tapentadol 1,3556 mmol entsprechen. In anderen Worten: die pharmazeutische Darreichungsform enthält eine moläquivalente Dosis von Tapentadol im Bereich von 0,0452 bis 1,3556 mmol.

Das Hydrochloridsalz von Tapentadol (Anhydrat) hat ein Molekulargewicht von 257,8 g/mol, während das Weinsäuresalz von Tapentadol (Anhydrat) ein Molekulargewicht von 371,4 g/mol hat. Somit enthält, wenn die erfindungsgemäße pharmazeutische Darreichungsform z. B. eine gewichtsäquivalente Dosis von 100 mg bezogen auf die freie Base von Tapentadol (0,4518 mmol) enthält, sie eigentlich 167,8 mg des Weinsäuresalzes von Tapentadol (0,4518 mmol).

Wenn nicht ausdrücklich anders angegeben, wird die Dosisinformation für Tapentadol somit als Äquivalentgewicht bezogen auf die freie Base an Tapentadol, d. h. die Nichtsalzform, die Nichtsolvatform, die Nichtkokristallform und die Nichtaggregatform von Tapentadol mit beliebigen anderen Molekülen ausgedrückt. Es wird in Betracht gezogen, dass die eigentlich in der pharmazeutischen Darreichungsform enthaltene Form von Tapentadol, d. h. das Salz von Tapentadol mit Weinsäure, insbesondere L-(+)-Weinsäure, in Form einer beliebigen polymorphen Form und/oder eines beliebigen Solvats und/oder eines beliebigen Kokristalls und/oder eines beliebigen anderen Aggregats eines solchen Salzes mit anderen Molekülen vorliegen kann.

Wenn nicht ausdrücklich anders angegeben, sind alle Prozentzahlen Gewichtsprozent und beziehen sich auf das Gesamtgewicht der pharmazeutischen Darreichungsform. Ist die pharmazeutische Darreichungsform beschichtet, so ist das Gewicht des Überzugs im Gesamtgewicht der pharmazeutischen Darreichungsform eingeschlossen.

Die erfindungsgemäße pharmazeutische Darreichungsform kann neben Tapentadol zusätzliche pharmakologische Wirkstoffe enthalten; jedoch ist Tapentadol vorzugsweise der einzige in der pharmazeutischen Darreichungsform enthaltene pharmakologische Wirkstoff.

Vorzugsweise ist Tapentadol homogen über die erfindungsgemäße pharmazeutische Darreichungsform verteilt.

Die erfindungsgemäße pharmazeutische Darreichungsform ist für die orale Verabreichung vorgesehen, vorzugsweise durch Schlucken der pharmazeutischen Darreichungsform als Ganzes. Die erfindungsgemäße pharmazeutischen Darreichungsform ist somit vorzugsweise nicht für die bukkale oder sublinguale Verabreichung vorgesehen, bei der die pharmazeutische Darreichungsform in der Mundhöhle verbleiben würde.

Die erfindungsgemäße pharmazeutische Darreichungsform ist für die zweimal tägliche Verabreichung vorgesehen. Die erfindungsgemäße pharmazeutische Darreichungsform enthält somit 50% der für die Verabreichung zum Herbeiführen der gewünschten therapeutischen Wirkung vorgesehenen Tagesdosis an Tapentadol.

Die zweimal tägliche Verabreichung kann in Abständen von etwa allen 12 Stunden erfolgen, wenngleich ein solches Protokoll nicht streng befolgt werden muss. Für die Zwecke der Beschreibung soll zweimal täglich auch ein Verabreichungsprotokoll umfassen, bei dem die beiden erfindungsgemäßen pharmazeutischen Darreichungsformen während eines Zeitraums von etwa 24 Stunden verabreicht werden, wobei die beiden Verabreichungen mindestens 4 Stunden getrennt voneinander erfolgen müssen, vorzugsweise mindestens 8 Stunden.

Die erfindungsgemäße pharmazeutische Darreichungsform stellt eine verlängerte Freisetzung von Tapentadol bereit. Für die Zwecke der Beschreibung bedeutet verlängerte Freisetzung eine nicht sofortige Freisetzung. Die verlängerte Freisetzung schließt die kontrollierte Freisetzung, die verzögerte Freisetzung, die retardierte Freisetzung, die stufenweise Freisetzung, die wiederholte Freisetzung, die anhaltende Freisetzung und die gleichmäßig anhaltende Freisetzung ein. Verlängerte Freisetzung bedeutet vorzugsweise eine Freisetzung mit einer reduzierten Freisetzungsrate, zum Aufrechterhalten einer therapeutischen Wirkung, zur Verminderung von toxischen Effekten oder für einen anderen therapeutischen Zweck.

Die verlängerte Freisetzung kann auf verschiedenen dem Fachmann bekannten Techniken beruhen. Gemäß einer bevorzugten Ausführungsform basiert die verlängerte Freisetzung auf Überzugsmaterialien mit verlängerter Freisetzung, mit denen die pharmazeutische Darreichungsform als solche oder mit denen eine Vielzahl von Partikeln beschichtet sein kann/können. Gemäß einer anderen bevorzugten Ausführungsform basiert die verlängerte Freisetzung auf einer Matrix mit verlängerter Freisetzung, in der Tapentadol vorzugsweise eingebettet ist. Erfindungsgemäß wird weiterhin in Betracht gezogen, dass sich eine verlängerte Freisetzung durch alternative Konzepte wie Ionenaustauscherharze, osmotische Darreichungsformen und dergleichen erzielen lässt.

Die erfindungsgemäße pharmazeutische Darreichungsform stellt nach oraler Verabreichung vorzugsweise Plasmaspiegel von Tapentadol bereit, die über eine Dauer von mindestens 6 Stunden, vorzugsweise mindestens 8 Stunden, besonders bevorzugt mindestens 10 Stunden, am meisten bevorzugt mindestens 12 Stunden Schmerzlinderung (Analgesie) bereitstellen.

Die erfindungsgemäße pharmazeutische Darreichungsform stellt vorzugsweise ein In-vitro-Auflösungsprofil bereit, bei dem, gemessen nach der USP-Paddelmethode bei 50 U/min in 900 ml wässrigem Phosphatpuffer bei einem pH-Wert von 6,8 bei 37°C,
- nach 0,5 Stunden 20±20 Gew.-%; vorzugsweise 20±15 Gew.-%; besonders bevorzugt 20±10 Gew.-%;
- nach 4 Stunden 60±20 Gew.-%; vorzugsweise 60±15 Gew.-%; besonders bevorzugt 60±10 Gew.-% und
- nach 12 Stunden mindestens 60 Gew.-%; vorzugsweise mindestens 70 Gew.-%; besonders bevorzugt mindestens 80 Gew.-%
des ursprünglich in der Darreichungsform enthaltenen Tapentadol freigesetzt worden sind.

Die erfindungsgemäße pharmazeutische Darreichungsform stellt vorzugsweise ein In-vitro-Auflösungsprofil bereit, bei dem, gemessen nach der USP-Paddelmethode bei 50 U/min in 900 ml wässrigem Phosphatpuffer bei einem pH-Wert von 6,8 bei 37°C,
- nach 1 Stunde 25±15 Gew.-%; vorzugsweise 25±10 Gew.-%; besonders bevorzugt 25±5,0 Gew.-%;
- nach 2 Stunden 35±20 Gew.-%; vorzugsweise 35±10 Gew.-%; besonders bevorzugt 35±5,0 Gew.-%;
- nach 4 Stunden 50±20 Gew.-%; vorzugsweise 50±10 Gew.-%; besonders bevorzugt 50±5,0 Gew.-%;
- nach 8 Stunden 80±20 Gew.-%; vorzugsweise 80±10 Gew.-%; besonders bevorzugt 80±5,0 Gew.-%;
des ursprünglich in der Darreichungsform enthaltenen Tapentadol freigesetzt worden sind.

Die erfindungsgemäße pharmazeutische Darreichungsform stellt vorzugsweise ein In-vitro-Auflösungsprofil bereit, bei dem, gemessen nach der USP-Paddelmethode bei 50 U/min in 900 ml wässrigem Puffer bei einem pH-Wert von 4,5 bei 37°C,
- nach 0,5 Stunden 20±20 Gew.-%; vorzugsweise 20±15 Gew.-%; besonders bevorzugt 20±10 Gew.-%;
- nach 4 Stunden 60±20 Gew.-%; vorzugsweise 60±15 Gew.-%; besonders bevorzugt 60±10 Gew.-% und
- nach 12 Stunden mindestens 60 Gew.-%; vorzugsweise mindestens 70 Gew.-%; besonders bevorzugt mindestens 80 Gew.-%
des ursprünglich in der Darreichungsform enthaltenen Tapentadol freigesetzt worden sind.

Die erfindungsgemäße pharmazeutische Darreichungsform stellt vorzugsweise ein In-vitro-Auflösungsprofil bereit, bei dem, gemessen nach der USP-Paddelmethode bei 50 U/min in 900 ml wässrigem Puffer bei einem pH-Wert von 4,5 bei 37°C,
- nach 1 Stunde 25±15 Gew.-%; vorzugsweise 25±10 Gew.-%; besonders bevorzugt 25±5,0 Gew.-%;
- nach 2 Stunden 35±20 Gew.-%; vorzugsweise 35±10 Gew.-%; besonders bevorzugt 35±5,0 Gew.-%;
- nach 4 Stunden 50±20 Gew.-%; vorzugsweise 50±10 Gew.-%; besonders bevorzugt 50±5,0 Gew.-%;
- nach 8 Stunden 80±20 Gew.-%; vorzugsweise 80±10 Gew.-%; besonders bevorzugt 80±5,0 Gew.-%;
des ursprünglich in der Darreichungsform enthaltenen Tapentadol freigesetzt worden sind.

Die erfindungsgemäße pharmazeutische Darreichungsform stellt vorzugsweise ein In-vitro-Auflösungsprofil bereit, bei dem, gemessen nach der USP-Paddelmethode bei 50 U/min in 900 ml 0,1 N HCl bei einem pH-Wert von 1,0 und 37 °C,
- nach 0,5 Stunden 20±20 Gew.-%; vorzugsweise 20±15 Gew.-%; besonders bevorzugt 20±10 Gew.-%;
- nach 4 Stunden 60±20 Gew.-%; vorzugsweise 60±15 Gew.-%; besonders bevorzugt 60±10 Gew.-% und
- nach 12 Stunden mindestens 60 Gew.-%; vorzugsweise mindestens 70 Gew.-%; besonders bevorzugt mindestens 80 Gew.-%
des ursprünglich in der Darreichungsform enthaltenen Tapentadol freigesetzt worden sind.

Die erfindungsgemäße pharmazeutische Darreichungsform stellt vorzugsweise ein In-vitro-Auflösungsprofil bereit, bei dem, gemessen nach der USP-Paddelmethode bei 50 U/min in 900 ml 0,1 N HCl bei einem pH-Wert von 1,0 und 37 °C,
- nach 1 Stunde 25±10 Gew.-%; vorzugsweise 25±10 Gew.-%; besonders bevorzugt 25±5,0 Gew.-%;
- nach 2 Stunden 40±30 Gew.-%; vorzugsweise 40±10 Gew.-%; besonders bevorzugt 40±5,0 Gew.-%;
- nach 4 Stunden 60±20 Gew.-%; vorzugsweise 60±10 Gew.-%; besonders bevorzugt 60±5,0 Gew.-%;
- nach 8 Stunden 80±20 Gew.-%; vorzugsweise 80±10 Gew.-%; besonders bevorzugt 80±5,0 Gew.-%;
des ursprünglich in der Darreichungsform enthaltenen Tapentadol freigesetzt worden sind.

Die erfindungsgemäße pharmazeutische Darreichungsform stellt vorzugsweise Resistenz gegen ethanolinduziertes Dose-Dumping bereit.

Die erfindungsgemäße pharmazeutische Darreichungsform stellt vorzugsweise eine langsamere In-vitro-Auflösung von Tapentadol in wässrigem ethanolhaltigem Medium als in nicht ethanolhaltigem Medium bereit.

Die erfindungsgemäße pharmazeutische Darreichungsform stellt vorzugsweise eine langsamere Auflösung von Tapentadol in 0,1 N HCl mit 5,0 Vol.-% Ethanol (pH 1,0) als in 0,1 N HCl ohne 5,0 Vol.-% Ethanol (pH 1,0) bereit, jeweils gemessen nach der USP-Paddelmethode bei 50 U/min in 900 ml bei 37°C.

Die erfindungsgemäße pharmazeutische Darreichungsform stellt vorzugsweise eine langsamere Auflösung von Tapentadol in 0,1 N HCl mit 20 Vol.-% Ethanol (pH 1,0) als in 0,1 N HCl ohne 20 Vol.-% Ethanol (pH 1,0) bereit, jeweils gemessen nach der USP-Paddelmethode bei 50 U/min in 900 ml bei 37°C.

Die erfindungsgemäße pharmazeutische Darreichungsform stellt vorzugsweise eine langsamere Auflösung von Tapentadol in 0,1 N HCl mit 40 Vol.-% Ethanol (pH 1,0) als in 0,1 N HCl ohne 40 Vol.-% Ethanol (pH 1,0) bereit, jeweils gemessen nach der USP-Paddelmethode bei 50 U/min in 900 ml bei 37°C.

Gemäß bevorzugten Ausführungsformen mit der erfindungsgemäßen pharmazeutischen Darreichungsform beträgt die gewichtsäquivalente Dosis von in der pharmazeutischen Darreichungsform enthaltenem Tapentadol 25 mg, 50 mg oder 100 mg, jeweils bezogen auf die freie Base von Tapentadol. Die erfindungsgemäße pharmazeutische Darreichungsform hat vorzugsweise ein Gesamtgewicht im Bereich von 150 bis 750 mg.

Gemäß bevorzugten Ausführungsformen mit der erfindungsgemäßen pharmazeutischen Darreichungsform beträgt die gewichtsäquivalente Dosis von in der pharmazeutischen Darreichungsform enthaltenem Tapentadol 150 mg, 200 mg oder 250 mg, jeweils bezogen auf die freie Base von Tapentadol. Die erfindungsgemäße pharmazeutische Darreichungsform hat vorzugsweise ein Gesamtgewicht im Bereich von 300 bis 1200 mg.

Die erfindungsgemäße pharmazeutische Darreichungsform umfasst vorzugsweise einen, zwei oder mehr physiologisch unbedenkliche Exzipienten.

Pharmazeutische Exzipienten sind dem Fachmann bekannt (vgl z. B. R. C. Rowe et al., Handbook of Pharmaceutical Excipients, Pharmaceutical Press; 6. Auflage 2009; E.-M. Hoepfner et al., Fiedler-Encyclopedia of Excipients, Editio Cantor, 6. Auflage 2008). Für die Zwecke der Beschreibung wird ein "pharmazeutischer Exzipient" vorzugsweise als eine beliebige pharmakologisch inaktive Substanz angesehen, die typischerweise als Träger für die Wirkstoffe eines Medikaments verwendet wird. Der pharmazeutische Exzipient kann eine physiologische Wirkung haben, z. B. wie ein Vitamin, jedoch keine pharmakologische Wirkung wie ein Arzneimittel. Typische Beispiele für pharmazeutische Exzipienten schließen Antihaftmittel, Bindemittel, Überzugsmaterialien, Sprengmittel, Füllstoffe, Verdünnungsmittel, Geschmackstoffe, Farbstoffe, Gleitmittel, Schmiermittel, Konservierungsstoffe, Sorbentien, Tenside, Süßstoffe, Färbemittel, Pigmente und dergleichen ein.

Beliebige der obigen Exzipienten können jeweils in Untergruppen eingeteilt werden. Konservierungsstoffe beispielsweise können in Antioxidationsmittel, Puffer, antimikrobielle Substanzen und dergleichen unterteilt werden, während Bindemittel in Lösungsbindemittel und Trockenbindemittel unterteilt werden können. Mehrere Exzipienten zeigen gleichzeitig verschiedene Eigenschaften, so dass sie verschiedenen Zwecken dienen können. Polyethylenglykol beispielsweise kann als Bindemittel, Weichmacher und dergleichen eingesetzt werden.

Die erfindungsgemäße pharmazeutische Darreichungsform, vorzugsweise die Matrix mit verlängerter Freisetzung, umfasst vorzugsweise ein Bindemittel.

Das Bindemittel ist vorzugsweise aus der aus Cellulose, Magnesiumaluminiumsilicaten (z. B. Bentonit), Mono-, Oligo- und Polysacchariden (z. B. Dextrose, Lactose, Mannose), Zuckeralkoholen (z. B. Lactit, Mannit), Stärken (z. B. vorgelatinisierter Stärke, hydrolysierter Stärke, modifizierter Stärke), Calciumphosphat, Polyvinylpyrrolidon und Vinylpyrrolidon-Vinylacetat-Copolymeren; vorzugsweise mikrokristalliner Cellulose; besonders bevorzugt silifizierter mikrokristalliner Cellulose, bestehenden Gruppe ausgewählt.

Der Inhalt an Bindemittel nach Gewicht beträgt vorzugsweise mindestens 5,0 Gew.-%, besonders bevorzugt mindestens 10 Gew.-%, noch mehr bevorzugt mindestens 15 Gew.-%, noch mehr bevorzugt mindestens 20 Gew.-%, noch mehr bevorzugt mindestens 25 Gew.-%, am meisten bevorzugt mindestens 30 Gew.-% und insbesondere mindestens 35 Gew.-%, jeweils bezogen auf das Gesamtgewicht der pharmazeutischen Darreichungsform.

Der Inhalt an Bindemittel nach Gewicht beträgt vorzugsweise höchstens 85 Gew.-%, besonders bevorzugt höchstens 82,5 Gew.-%, noch mehr bevorzugt höchstens 80 Gew.-%, noch mehr bevorzugt höchstens 77,5 Gew.-%, noch mehr bevorzugt höchstens 75 Gew.-%, am meisten bevorzugt höchstens 72,5 Gew.-% und insbesondere höchstens 70 Gew.-%, jeweils bezogen auf das Gesamtgewicht der pharmazeutischen Darreichungsform.

Der Inhalt an Bindemittel nach Gewicht liegt im Bereich von 52±30 Gew.-%, besonders bevorzugt von 52=L27,5 Gew.-%, noch mehr bevorzugt von 52:L25 Gew.-%, noch mehr bevorzugt von 52=L22,5 Gew.-%, noch mehr bevorzugt von 52±20 Gew.-%, am meisten bevorzugt von 52±17,5 Gew.-% und insbesondere von 52±15 Gew.-%, jeweils bezogen auf das Gesamtgewicht der pharmazeutischen Darreichungsform.

Enthält die pharmazeutische Darreichungsform mehr als ein Bindemittel, so beziehen sich die obigen Prozentzahlen auf den Gesamtgehalt aller in der pharmazeutischen Darreichungsform enthaltenen Bindemittel.

Die erfindungsgemäße pharmazeutische Darreichungsform umfasst vorzugsweise ein Schmiermittel.

Das Schmiermittel ist vorzugsweise aus der aus Salzen von Fettsäuren (z. B. Magnesiumstearat, Calciumstearat, Zinkstearat), Fettsäuren (z. B. Stearinsäure, Palmitinsäure), Glycerylfettsäureestern (z. B. Glycerylmonostearat, Glycerylmonobehenat, Glyceryldibehenat, Glyceryltribehenat), Sorbitanmonostearat, Saccharosemonopalmitat, Natriumstearylfumarat, hydratisiertem Magnesiumsilicat und Talk bestehenden Gruppe ausgewählt und ist vorzugsweise Magnesiumstearat.

Der Inhalt an Schmiermittel nach Gewicht beträgt mindestens 0,20 Gew.-%, besonders bevorzugt mindestens 0,25 Gew.-%, noch mehr bevorzugt mindestens 0,30 Gew.-%, noch mehr bevorzugt mindestens 0,35 Gew.-%, noch mehr bevorzugt mindestens 0,40 Gew.-%, am meisten bevorzugt mindestens 0,45 Gew.-% und insbesondere mindestens 0,50 Gew.-%, jeweils bezogen auf das Gesamtgewicht der pharmazeutischen Darreichungsform.

Der Inhalt an Schmiermittel nach Gewicht beträgt höchstens 3,0 Gew.-%, besonders bevorzugt höchstens 2,8 Gew.-%, noch mehr bevorzugt höchstens 2,6 Gew.-%, noch mehr bevorzugt höchstens 2,40 Gew.-%, noch mehr bevorzugt höchstens 2,20 Gew.-%, am meisten bevorzugt höchstens 2,00 Gew.-% und insbesondere höchstens 1,80 Gew.-%, jeweils bezogen auf das Gesamtgewicht der pharmazeutischen Darreichungsform.

Der Inhalt an Schmiermittel nach Gewicht liegt vorzugsweise im Bereich von 0,1±1,0 Gew.-%, besonders bevorzugt von 0,50±0,45 Gew.-%, noch mehr bevorzugt von 0,50±0,40 Gew.-%, noch mehr bevorzugt von 0,50±0,35 Gew.-%, noch mehr bevorzugt von 0,50±0,30 Gew.-%, am meisten bevorzugt von 0,50±0,25 Gew.-% und insbesondere von 0,50±0,20 Gew.-%, jeweils bezogen auf das Gesamtgewicht der pharmazeutischen Darreichungsform.

Gemäß bevorzugten Ausführungsformen der erfindungsgemäßen pharmazeutischen Darreichungsform enthält die Darreichungsform einen Überzug mit verlängerter Freisetzung, der ein Überzugsmaterial mit verlängerter Freisetzung ausgewählt aus der Gruppe bestehend aus hydrophoben Celluloseethern, Acrylpolymeren, Schellack, Zein, hydrophoben wachsartigen Produkten und Mischungen davon umfasst.

Es wird in Betracht gezogen, dass es sich bei der Darreichungsform um einen Monolith handelt, der einen solchen Überzug mit verlängerter Freisetzung umfasst. Gemäß einer bevorzugten Ausführungsform ist die Darreichungsform multipartikulär, wobei die einzelnen Partikel (Granulate, Pellets und dergleichen) einen solchen Überzug mit verlängerter Freisetzung umfassen.

Die Anzahl an in der Darreichungsform enthaltenen Partikeln ist nicht besonders eingeschränkt und kann im Bereich von 1, 2, 3, 4 oder 5 bis 10, 20, 30, 40 bis 100, 200 und mehr liegen.

Die Partikel haben vorzugsweise im Wesentlichen das gleiche Gewicht, die gleiche Größe und gleiche Zusammensetzung.

Gemäß einer bevorzugten Ausführungsform enthalten die Partikel einen Kern, der im Wesentlichen die Gesamtmenge an Tapentadol umfasst, gegebenenfalls zusammen mit einem oder mehreren Exzipienten, und einen den Kern verkapselnden Überzug mit verlängerter Freisetzung.

Gemäß einer anderen bevorzugten Ausführungsform enthalten die Partikel einen inerten Kern, der kein Tapentadol enthält (z. B. Zuckerkügelchen), eine Arzneimittelüberzugsschicht, die den Kern verkapselt und im Wesentlichen die gesamte Menge an Tapentadol umfasst, gegebenenfalls zusammen mit einem oder mehreren Exzipienten, und einen den Kern und die Arzneimittelüberzugsschicht verkapselnden Überzug mit verlängerter Freisetzung.

Das Überzugsmaterial mit verlängerter Freisetzung ist vorzugsweise aus der aus Acrylsäure- und Methacrylsäure-Copolymeren, Aminoalkylmethacrylat-Copolymeren, Cyanoethylmethacrylaten, Ethoxyethylmethacrylaten, Ethylcellulose, Methacrylsäurealkylamid-Copolymeren, Methacrylsäure-Copolymeren, Methylmethacrylaten, Methylmethacrylat-Copolymeren, Poly(acrylsäure), Poly(methacrylsäure) (anhydrid), Poly(methacrylsäure), Glycidylmethacrylat-Copolymeren, Poly(methylmethacrylat), Poly(methylmethacrylat)-Copolymeren, Polyacrylamid und Polymethacrylat bestehenden Gruppe ausgewählt.

Gemäß bevorzugten Ausführungsformen besteht das Acrylpolymer aus einem oder mehreren Ammoniomethacrylatcopolymeren, d. h. Copolymeren von Acryl- und Methacrylsäureestern mit einem niedrigen Gehalt an quartären Ammoniumgruppen. Zu Erhalt eines wünschenswerten Auflösungsprofils kann es erforderlich sein, zwei oder mehr Ammoniomethacrylat-Copolymere mit unterschiedlichen physikalischen Eigenschaften wie unterschiedlichen molaren Verhältnissen an quartären Ammoniumgruppen zu neutralen (Meth)acrylsäureestern aufzunehmen.

Der Überzug wird vorzugsweise aus einer wässrigen Dispersion oder aus einer organischen Dispersion oder aus einer organischen Lösung eines hydrophoben Polymers hergestellt. Der Überzug umfasst vorzugsweise eine wirksame Menge eines Weichmachers, der auch in der wässrigen Dispersion des hydrophoben Polymers vorhanden ist. Der Weichmacher verbessert die physikalischen Eigenschaften des Films weiter. Da beispielsweise Ethylcellulose eine relativ hohe Glasübergangstemperatur aufweist und unter normalen Beschichtungsbedingungen keine flexiblen Filme ausbildet, ist es erforderlich, die Ethylcellulose vor deren Verwendung als Überzugsmaterial zu plastifizieren. Im Allgemeinen basiert die einer Überzugslösung zugesetzte Menge an Weichmacher auf der Konzentration des Filmbildners, z. B. am häufigsten von etwa 1 bis etwa 50 Gewichtsprozent des Filmbildners.

Beispiele für geeignete Weichmacher für Ethylcellulose schließen wasserunlösliche Weichmacher wie Dibutylsebacat, Diethylphthalat, Triethylcitrat, Tributylcitrat und Triacetin ein, obgleich es möglich ist, andere wasserunlösliche Weichmacher (wie acetylierte Monoglyceride, Phthalatester, Rhizinusöl usw.) zu verwenden. Triethylcitrat ist ein besonders bevorzugter Weichmacher für die wässrigen Dispersionen von Ethylcellulose der vorliegenden Erfindung.

Beispiele für geeignete Weichmacher für die Acrylpolymere der vorliegenden Erfindung schließen, wobei dies nicht einschränkend ist, Citronensäureester wie Triethylcitrat NF XVI, Tributylcitrat, Dibutylphthalat und gegebenenfalls 1,2-Propylenglykol ein. Andere Weichmacher, die sich als geeignet für die Verbesserung der Elastizität der von Acrylfilmen gebildeten Filme wie Eudragit^{®} RL/RS-Lacklösungen erwiesen haben, schließen Polyethylenglykole, Propylenglykole, Diethylphthalat, Rhizinusöl und Triacetin ein. Triethylcitrat ist ein besonders bevorzugter Weichmacher für die wässrigen Dispersionen von Ethylcellulose der vorliegenden Erfindung.

Die Zugabe von Talk reduziert die Neigung der wässrigen Dispersion zum Anhaften während der Verarbeitung und wirkt als Poliermittel.

Zusätzlich zum Modifizieren des Auflösungsprofils durch Veränderung der relativen Mengen der verschiedenen Acrylharzlacke lässt sich das Auflösungsprofil des letztendlich erhaltenen Produkts auch modifizieren, indem man beispielsweise die Dicke des retardierenden Überzugs erhöht oder reduziert. Verwendet man die wässrige Dispersion des hydrophoben Polymers zum Beschichten inerter pharmazeutischer Kügelchen, so kann man anschließend eine Vielzahl der erhaltenen stabilisierten festen Kügelchen mit verlängerter Freisetzung in einer Menge, die bei Einnahme und Kontakt mit Magensaft zur Bereitstellung einer wirksamen Dosis mit verlängerter Freisetzung ausreicht, in eine Gelatinekapsel geben.

Das Profil der verlängerten Freisetzung lässt sich beispielsweise durch Variieren der Menge an Ummantelung mit der wässrigen Dispersion des hydrophoben Polymers, der Veränderung der Weise, in der der Weichmacher der wässrigen Dispersion des hydrophoben Polymers zugesetzt wird, durch Variieren der Menge an Weichmacher relativ zum hydrophoben Polymer, durch Aufnahme zusätzlicher Inhaltsstoffe oder Exzipienten, durch Veränderung des Herstellungsverfahrens usw. verändern.

Der Überzug enthält vorzugsweise zusätzlich zum Filmbildner, Weichmacher und Lösungsmittelsystem (d. h. Wasser) einen Farbstoff zur Bereitstellung von Eleganz und Produktabgrenzung. Geeignete Inhaltsstoffe für die Bereitstellung von Farbe für die Formulierung bei Verwendung einer wässrigen Dispersion eines Acrylpolymers schließen Titandioxid und Farbpigmente wie Eisenoxidpigmente ein. Die Aufnahme von Pigmenten kann jedoch die retardierende Wirkung des Überzugs erhöhen.

Die plastizierte wässrige Dispersion oder organische Dispersion oder organische Lösung des hydrophoben Polymers kann durch Sprühen mittels eines beliebigen im Stand der Technik bekannten geeigneten Sprühgeräts auf das Tapentadol umfassende Substrat aufgebracht werden. Bei einem bevorzugten Verfahren verwendet man ein Wurster-Wirbelschichtsystem, bei dem ein von unten eingeführter Luftstrahl das Kernmaterial fluidisiert und beim Aufsprühen des Acrylpolymerüberzugs eine Trocknung bewirkt. Vorzugsweise bringt man eine zum Erhalt einer vorbestimmten kontrollierten Freisetzung von Tapentadol bei Exposition des überzogenen Substrats mit wässrigen Lösungen, z. B. Magensaft, ausreichende Menge der wässrigen Dispersion des hydrophoben Polymers auf, unter Berücksichtigung der Weise, in der der Weichmacher eingearbeitet wurde, usw. Nach dem Beschichten mit dem hydrophoben Polymer kann man gegebenenfalls eine weitere Ummantelung eines Filmbildners wie Opadry^{®} auf die Kügelchen aufbringen. Diese Umwandlung wird, wenn überhaupt, nur bereitgestellt, um die Agglomeration der Kügelchen wesentlich zu verringern.

Die Freisetzung von Tapentadol aus der Formulierung mit verlängerter Freisetzung lässt sich weiterhin durch Zugabe eines oder mehrerer freisetzungsmodifizierender Mittel oder durch Bereitstellung einer oder mehrerer Passagen durch den Überzug beeinflussen, d. h. auf eine gewünschte Rate einstellen. Das Verhältnis von hydrophobem Polymer zu wasserlöslichem Material wird neben anderen Faktoren durch die erforderliche Freisetzungsrate und die Löslichkeitscharakteristika der gewählten Materialien bestimmt. Die als Porenbildner fungierenden freisetzungsmodifizierenden Mittel können organisch oder anorganisch sein und Materialien einschließen, die in der Anwendungsumgebung aus dem Überzug herausgelöst, extrahiert oder ausgelaugt werden können. Die Porenbildner können ein oder mehrere hydrophile Polymere wie Hydroxypropylmethylcellulose umfassen. Die Überzüge für die kontrollierte Freisetzung können außerdem erosionsfördernde Mittel wie Stärken und Gummen einschließen. Die Überzüge für die kontrollierte Freisetzung gemäß der vorliegenden Erfindung können außerdem Materialien einschließen, die sich zur Bildung von mikroporösen Lamina in der Anwendungsumgebung eignen, wie aus geradkettigen Kohlensäurepolyestern bestehende Polycarbonate, bei denen die Carbonatgruppen in der Polymerkette wiederauftreten.

Gemäß einer bevorzugten Ausführungsform ist die Darreichungsform multipartikulär, wobei die individuellen Partikel (Granulate, Pellets und dergleichen) mit einem Überzug für eine verlängerte Freisetzung in einer Kapsel enthalten sind, gegebenenfalls zusammen mit zusätzlichen Exzipienten, die in der Kapsel im Pulverform oder ebenfalls in Form von Partikeln (Granulate, Pellets und dergleichen) enthalten sein können. Im letzteren Fall enthalten die Kapseln mindestens zwei verschiedene Typen von Partikeln, nämlich Partikel, die Tapentadol enthalten, und Partikel, die kein Tapentadol enthalten.

Gemäß einer anderen bevorzugten Ausführungsform ist die Darreichungsform multipartikulär, wobei die individuellen Partikel (Granulate, Pellets und dergleichen) mit einem Überzug für eine verlängerte Freisetzung in einer Tablette enthalten sind, die ein extrapartikuläres Material enthält (Multiple Unit Pellet System, MUPS). Das extrapartikuläre Material enthält vorzugsweise mindestens einen Exzipienten ausgewählt aus Bindemitteln, Sprengmitteln und Schmiermitteln.

Gemäß bevorzugten Ausführungsformen der erfindungsgemäßen pharmazeutischen Darreichungsform ist das Tapentadol in einer Matrix mit verlängerter Freisetzung eingebettet.

Vorzugsweise enthält die erfindungsgemäße pharmazeutische Darreichungsform, vorzugsweise die Matrix mit verlängerter Freisetzung, mindestens ein physiologisch unbedenkliches Polymer, das zur Verzögerung der Freisetzung des pharmakologischen Wirkstoffs aus der pharmazeutischen Darreichungsform dient. Das mindestens eine physiologisch unbedenkliche Polymer ist somit Teil der Matrix für die verlängerte Freisetzung der erfindungsgemäßen pharmazeutischen Darreichungsform.

Die Matrix mit verlängerter Freisetzung umfasst vorzugsweise mindestens ein Matrixmaterial mit verlängerter Freisetzung ausgewählt aus der Gruppe bestehend aus hydrophilen oder hydrophoben Polymeren und Kohlenwasserstoffen oder besteht im Wesentlichen daraus.

Gemäß bevorzugten Ausführungsformen der erfindungsgemäßen pharmazeutischen Darreichungsform umfasst die Matrix mit verlängerter Freisetzung mindestens ein Polymer ausgewählt aus der Gruppe bestehend aus bzw. besteht die Matrix mit verlängerter Freisetzung im Wesentlichen aus mindestens einem Polymer ausgewählt aus der Gruppe bestehend aus
- Polysacchariden oder Gummen (z. B. Xanthan, Guaran, Karayagummi, Johannisbrotkernmehl, Natriumalginat, Karobgummi, Chitosan, Polysacchariden von Mannose und Galactose, Pektin, Tragant, Agar);
- Celluloseethern (z. B. HPMC, HPC, HEC, MC, EC);
- Celluloseestern (z. B. Celluloseacetat, Celluloseacetatsuccinat, Celluloseacetatphthalat, Celluloseacetatbutyrat);
- Polyalkylenglykolen und Polyalkylenoxiden;
- Polyvinylalkohol (PVA), quervernetztem Polyvinylalkohol (PVA), Polyvinylpyrrolidon (PVP), quervernetztem Polyvinylpyrrolidon (PVP), Polyvinylpyrrolidon-Vinylacetat-Copolymeren, Polyvinylchlorid (PVC), Polyethylenvinylacetat (PVAc), Polydimethylsiloxan (PDS), Polyetherurethan (PEU), Polylmilchsäure (PLA), Polyglykolsäure (PGA), Polycaprolacton (PCL), Polyanhydriden, Polyorthoestern;
- Acrylharzen (z.B. quervernetzten Homopolymeren und Copolymeren von Acrylsäuren, Acrylsäure- und Methacrylsäure-Copolymeren, Aminoalkylmethacrylat-Copolymer, Cyanoethylmethacrylat, Ethoxyethylmethacrylaten, Methacrylsäurealkylamid-Copolymer, Methacrylsäure-Copolymeren, Methylmethacrylaten, Methylmethacrylat-Copolymeren, Poly(acrylsäure), Poly(methacrylsäure), Poly(methacrylsäureanhydrid), Glycidylmethacrylat-Copolymeren, Poly(methylmethacrylat), Poly(methylmethacrylat)-Copolymeren, Polyacrylamid, Polyhydroxyethylmethacrylat (PHEMA) und Polymethacrylat); und
- aus Proteinen gewonnenen Materialien.

Vorzugsweise umfasst die Matrix mit verlängerter Freisetzung mindestens einen Kohlenwasserstoff ausgewählt aus der Gruppe bestehend aus langkettigen (C₈-C₅₀, insbesondere C₁₂-C₄₀) Fettsäuren, langkettigen Fettalkoholen, Glycerylestern von langkettigen Fettsäuren, Mineralölen, Pflanzenölen und Wachsen oder besteht im Wesentlichen daraus.

Die Matrix mit verlängerter Freisetzung umfasst vorzugsweise ein Matrixmaterial mit verlängerter Freisetzung ausgewählt aus der Gruppe bestehend aus (i) Hydroxypropylmethylcellulose (HPMC); (ii) Hydroxypropylcellulose (HPC); (iii) Hydroxyethylcellulose (HEC); (iv) mikrokristalliner Cellulose (MCC); (v) Ethylcellulose (EC); (vi) Polyvinylacetat (PVAc); (vii) Polyvinylpyrrolidon (PVP); (viii) Polyvinylpyrrolidon-Vinylacetat-Copolymer (PVP/PVAc); (ix) Poly(ethylacrylat-co- methylmethacrylat-co-trimethylammonioethylmethacrylatchlorid); (x) Poly(butylmethacrylat-co-(2-dimethylaminoethyl)methacrylat-co-methylmethacrylat); (xi) Poly(methylmethacrylat-co-methacrylsäure); (xii) Poly(ethylacrylat-co-methacrylsäure); (xiii) Poly(methylacrylat-co-methylmethacrylat-co-methacrylsäure); (xiv) Poly(ethylacrylat-co-methylmethacrylat); (xv) Poly(ethylenoxid) (PEO); (xvi) Polyethylenglykol (PEG); (xvii) langkettigem Fettalkohol mit 8 bis 50 Kohlenstoffatomen, vorzugsweise 12 bis 40 Kohlenstoffatomen, der gesättigt oder ungesättigt und geradkettig oder verzweigt sein kann; (xviii) Cetostearylalkohol; (xix) Stearylalkohol; (xx) Cetylalkohol; (xxi) Kohlenwasserstoff ausgewählt aus der Gruppe bestehend aus langkettigen Fettsäuren mit 8 bis 50 Kohlenstoffatomen, vorzugsweise 12 bis 40 Kohlenstoffatomen, der gesättigt oder ungesättigt und geradkettig oder verzweigt sein kann; Glycerylestern solcher langkettigen Fettsäuren, Mineralölen, Pflanzenölen und Wachsen; (xxii) Xanthan; (xxiii) Natriumalginat; (xxiv) Guaran; (xxv) Johannisbrotkernmehl; und beliebigen Mischungen der obigen, oder besteht im Wesentlichen daraus. Der Gehalt an Matrixmaterial mit verlängerter Freisetzung liegt vorzugsweise im Bereich von 15±10 Gew.-% oder 20±10 Gew.-% oder 25±10 Gew.-% oder 30±10 Gew.-% oder 35±10 Gew.-% oder 40±10 Gew.-% oder 45±10 Gew.-% oder 50±10 Gew.-% oder 55±10 Gew.-% oder 60±10 Gew.-% oder 65±10 Gew.-% oder 70±10 Gew.-% oder 75±10 Gew.-% oder 80±10 Gew.-%, jeweils bezogen auf das Gesamtgewicht der Darreichungsform.

Die erfindungsgemäßen pharmazeutischen Darreichungsformen umfassen vorzugsweise weder Poly(alkylenoxid), z. B. Poly(ethylenoxid), noch Ethylen-Vinylacetat-Copolymere (EVA). Für die Zwecke der Beschreibung unterscheidet sich Poly(alkylenoxid) von Poly(alkylenglykol) durch sein Molekulargewicht; Polymere mit einem gewichtsmittleren Molekulargewicht M_{w} von weniger als 100000 g/mol werden als Poly(alkylenglykol)angesehen, während Polymere mit einem gewichtsmittleren Molekulargewicht M_{w} von 100000 g/mol oder mehr als Poly(alkylenoxid) angesehen werden.

Die Matrix mit verlängerter Freisetzung umfasst vorzugsweise ein Cellulosederivat ausgewählt aus Celluloseethern und Celluloseestern oder ein Poly(meth)acrylat oder Copolymer davon.

Das Cellulosederivat ist vorzugsweise ein Celluloseether ausgewählt aus der Gruppe bestehend aus Methylcellulose, Ethylcellulose, Propylcellulose, Hydroxyethylcellulose, Hydroxypropylcellulose und Hydroxypropylmethylcellulose; bevorzugt Hydroxypropylmethylcellulose.

Bei dem Cellulosederivat handelt es sich bevorzugt um Hydroxypropylmethylcellulose. Die Hydroxypropylmethylcellulose ist vorzugsweise ausgewählt aus den Hypromellosetypen 1828, 2208, 2906 und 2910 gemäß USP mit dem folgenden Methoxylgehalt und Hydroxypropoxylgehalt:

| | Methoxyl (%) | | Hydroxypropoxyl (%) | |
|---|---|---|---|---|
| Typ | min | max | min | max |
| 1828 | 16,5 | 20,0 | 23,0 | 32,0 |
| 2208 | 19,0 | 24,0 | 4,0 | 12,0 |
| 2906 | 27,0 | 30,0 | 4,0 | 7,5 |
| 2910 | 28,0 | 30,0 | 7,0 | 12,0 |

Die Viskosität des physiologisch unbedenklichen Polymers, vorzugsweise Cellulosederivats, besonders bevorzugt Hydroxypropylmethylcellulose, liegt vorzugsweise im Bereich von 100000±80000 mPa·s, besonders bevorzugt 100000±60000 mPa·s, noch mehr bevorzugt 100000±40000 mPa·s, noch mehr bevorzugt 100000±20000 mPa·s.

Das zahlenmittlere Molekulargewicht Mₙ des physiologisch unbedenklichen Polymers, vorzugsweise Cellulosederivats, besonders bevorzugt Hydroxypropylmethylcellulose, beträgt vorzugsweise nicht mehr als 220000 g/mol, besonders bevorzugt nicht mehr als 180000 g/mol, noch mehr bevorzugt nicht mehr als 140000 g/mol, noch mehr bevorzugt nicht mehr als 120000 g/mol, noch mehr bevorzugt nicht mehr als 110000 g/mol, am meisten bevorzugt nicht mehr als 86000 g/mol und insbesondere nicht mehr als 63000 g/mol.

Der Inhalt an physiologisch unbedenklichem Polymer, vorzugsweise Celluloseether, nach Gewicht beträgt vorzugsweise mindestens 2,0 Gew.-%, besonders bevorzugt mindestens 3,0 Gew.-%, noch mehr bevorzugt mindestens 4,0 Gew.-%, noch mehr bevorzugt mindestens 5,0 Gew.-%, noch mehr bevorzugt mindestens 6,0 Gew.-%, am meisten bevorzugt mindestens 7,0 Gew.-% und insbesondere mindestens 8,0 Gew.-%, jeweils bezogen auf das Gesamtgewicht der pharmazeutischen Darreichungsform.

Der Inhalt an physiologisch unbedenklichem Polymer, vorzugsweise Celluloseether, nach Gewicht beträgt vorzugsweise höchstens 62,5 Gew.-%, besonders bevorzugt höchstens 60 Gew.-%, noch mehr bevorzugt höchstens 57,5 Gew.-%, noch mehr bevorzugt höchstens 55 Gew.-%, noch mehr bevorzugt höchstens 52,5 Gew.-%, am meisten bevorzugt höchstens 50 Gew.-% und insbesondere höchstens 47,5 Gew.-%, jeweils bezogen auf das Gesamtgewicht der pharmazeutischen Darreichungsform.

Der Inhalt an physiologisch unbedenklichem Polymer, vorzugsweise Celluloseether, nach Gewicht liegt vorzugsweise im Bereich von 30±28 Gew.-%, besonders bevorzugt von 30±26 Gew.-%, noch mehr bevorzugt von 30±24 Gew.-%, noch mehr bevorzugt von 30±22 Gew.-%, noch mehr bevorzugt von 30±20 Gew.-%, am meisten bevorzugt von 30±18 Gew.-% und insbesondere von 30±16 Gew.-%, jeweils bezogen auf das Gesamtgewicht der pharmazeutischen Darreichungsform.

Enthält die pharmazeutische Darreichungsform mehr als einen physiologisch unbedenklichen Polymer, der dem Zweck der signifikanten Retardierung der Freisetzung des pharmakologischen Wirkstoffs aus der pharmazeutischen Darreichungsform dient, vorzugsweise Celluloseether, so beziehen sich die obigen Prozentzahlen auf den Gesamtgehalt aller solcher physiologisch unbedenklichen Polymere, vorzugsweise Celluloseether, die in der pharmazeutischen Darreichungsform enthalten sind.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Hydroxypropylmethylcellulose (HPMC) umfasst, vorzugsweise in einer Menge von 5,0 bis 60 Gew.-%, z. B. 15±10 Gew.-% oder 20±10 Gew.-% oder 25±10 Gew.-% oder 30±10 Gew.-% oder 35±10Gew.-% oder 40±10 Gew.-% oder 45±10 Gew.-% oder 50±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Hydroxypropylcellulose (HPC) umfasst, vorzugsweise in einer Menge von 10 bis 50 Gew.-%, z. B. 20±10 Gew.-% oder 25±10 Gew.-% oder 30±10 Gew.-% oder 35±10 Gew.-% oder 40±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Hydroxyethylcellulose (HEC) umfasst, vorzugsweise in einer Menge von 5,0 bis 50 Gew.-%, z. B. 15±10 Gew.-% oder 20±10 Gew.-% oder 25±10 Gew.-% oder 30±10 Gew.-% oder 35±10 Gew.-% oder 40±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung mikrokristalline Cellulose (MCC) umfasst, vorzugsweise in einer Menge von 10 bis 70 Gew.-%, z. B. 20±10 Gew.-% oder 25±10 Gew.-% oder 30±10 Gew.-% oder 35±10Gew.-% oder 40±10 Gew.-% oder 45±10 Gew.-% oder 50±10 Gew.-% oder 55±10 Gew.-% oder 60±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Ethylcellulose (EC) umfasst, vorzugsweise in einer Menge von 5,0 bis 30 Gew.-%, z. B. 15±10 Gew.-% oder 20±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Polyvinylacetat (PVAc) umfasst, vorzugsweise in einer Menge von 25 bis 70 Gew.-%, z. B. 35±10Gew.-% oder 40±10 Gew.-% oder 45±10 Gew.-% oder 50±10 Gew.-% oder 55±10 Gew.-% oder 60±10 Gew.-%, jeweils bezogen auf das Gesamtgewicht der Darreichungsform. Das Polyvinylacetat wird vorzugsweise in Form einer Mischung mit Polyvinylpyrrolidon (Povidon) eingesetzt. Eine solche bevorzugte Mischung ist im Handel erhältlich, z. B. als Kollidon^{®} SR (80 Gew.-% Polyvinylacetat, 19 Gew.-% Povidon, 0,8 Gew.-% Natriumlaurylsulfat und 0,2 Gew.-% Kieselsäure).

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Polyvinylpyrrolidon (PVP) umfasst, vorzugsweise in einer Menge von 2,0 bis 21 Gew.-%, z. B. 10±5.0 Gew.-% oder 15±5,0 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Polyvinylpyrrolidon-Vinylacetat-Copolymer (PVP/PVAc) umfasst, vorzugsweise in einer Menge von 1,0 bis 30 Gew.-%, z. B. 15±10 Gew.-% oder 20±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Poly(ethylacrylat-co-methylmethacrylat-co-trimethylammonioethylmethacrylatchlorid) umfasst, vorzugsweise in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform. Poly(ethylacrylat-co-methylmethacrylat-co-trimethylammonioethylmethacrylatchlorid) ist im Handel z. B. als Eudragit^{®} RS und Eudragit^{®} RL erhältlich.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Poly(butylmethacrylat-co-(2-dimethylaminoethyl)methacrylat-co-methylmethacrylat) umfasst, vorzugsweise in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform. Poly(butylmethacrylat-co-(2-dimethylaminoethyl)methacrylat-co-methylmethacrylat) ist im Handel z. B. als Eudragit^{®} E erhältlich.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Poly(methylmethacrylate-co-methacrylsäure) umfasst, vorzugsweise in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform. Poly(methylmethacrylat-co-methacrylsäure) ist im Handel z. B. als Eudragit^{®} L erhältlich.

Gemäß einer besonders bevorzugten Ausfuhrungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Poly(ethylacrylat-co-methacrylsäure) umfasst, vorzugsweise in einer Menge von 5,0 bis 45 Gew.-%, z. B. 15±10 Gew.-% oder 20±10 Gew.-% oder 25±10 Gew.-% oder 30±10 Gew.-% oder 35±10Gew.-%, jeweils bezogen auf das Gesamtgewicht der Darreichungsform. Poly(ethylacrylat-co-methacrylsäure) ist im Handel z. B. als Eudragit^{®} S erhältlich.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Poly(methylacrylat-co-methylmethacrylat-co-methacrylsäure) umfasst, vorzugsweise in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform. Poly(methylacrylat-co-methylmethacrylat-co-methacrylsäure) ist im Handel z. B. als Eudragit^{®} FS erhältlich.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Poly(ethylacrylat-co-methylmethacrylat) umfasst, vorzugsweise in einer Menge von 5,0 bis 45 Gew.-%, z. B. 15±10 Gew.-% oder 20±10 Gew.-% oder 25±10 Gew.-% oder 30±10 Gew.-% oder 35±10Gew.-%, jeweils bezogen auf das Gesamtgewicht der Darreichungsform. Poly(ethylacrylat-co-methylmethacrylat) ist im Handel z. B. als Eudragit^{®} NE erhältlich.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Poly(ethylenoxid) (PEO) umfasst, vorzugsweise in einer Menge von 25 bis 65 Gew.-%, z. B. 35±10 Gew.-% oder 40±10 Gew.-% oder 45±10 Gew.-% oder 50±10 Gew.-% oder 55±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Polyethylenglykol (PEG) umfasst, vorzugsweise in einer Menge von 5,0 bis 35 Gew.-%, z. B. 15±10 Gew.-% oder 20±10 Gew.-% oder 25±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung einen langkettigen Fettalkohol mit 8 bis 50 Kohlenstoffatomen, vorzugsweise 12 bis 40 Kohlenstoffatomen, der gesättigt oder ungesättigt und geradkettig oder verzweigt sein kann, umfasst, vorzugsweise in einer Menge von 15 bis 40 Gew.-%, z. B. 25±10 Gew.-% oder 30±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Cetostearylalkohol umfasst, vorzugsweise in einer Menge von 15 bis 40 Gew.-%, z. B. 25±10 Gew.-% oder 30±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Stearylalkohol umfasst, vorzugsweise in einer Menge von 15 bis 40 Gew.-%, z. B. 25±10 Gew.-% oder 30±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Cetylalkohol umfasst, vorzugsweise in einer Menge von 15 bis 40 Gew.-%, z. B. 25±10 Gew.-% oder 30±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung einen Kohlenwasserstoff ausgewählt aus der Gruppe der langkettigen Fettsäuren mit 8 bis 50 Kohlenstoffatomen, vorzugsweise 12 bis 40 Kohlenstoffatomen, der gesättigt oder ungesättigt und geradkettig oder verzweigt sein kann, Glycerylester solcher langkettigen Fettsäuren, Mineralöle, Pflanzenöle und Wachse umfasst, jeweils vorzugsweise in einer Menge von 5,0 bis 70 Gew.-%, z. B. 15±10 Gew.-% oder 20±10 Gew.-% oder 25±10 Gew.-% oder 30±10 Gew.-% oder 35±10 Gew.-% oder 40±10 Gew.-% oder 45±10 Gew.-% oder 50±10 Gew.-% oder 55±10 Gew.-% oder 60±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Xanthan umfasst, vorzugsweise in einer Menge von 15 bis 40 Gew.-%, z. B. 25±10 Gew.-% oder 30±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Natriumalginat umfasst, vorzugsweise in einer Menge von 15 bis 40 Gew.-%, z. B. 25±10 Gew.-% oder 30±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Guaran umfasst, vorzugsweise in einer Menge von 5,0 bis 35 Gew.-%, z. B. 15±10 Gew.-% oder 20±10 Gew.-% oder 25±10 Gew.-%, jeweils bezogen auf das Gesamtgewicht der Darreichungsform.

Gemäß einer besonders bevorzugten Ausführungsform umfasst die erfindungsgemäße pharmazeutische Darreichungsform eine gewichtsäquivalente Dosis an Tapentadol in dem Bereich von 10 bis 300 mg, z. B. 25 mg, 50 mg, 100 mg, 150 mg, 200 mg oder 250 mg, jeweils bezogen auf die freie Base von Tapentadol, und eine Matrix mit verlängerter Freisetzung, in der das Salz von Tapentadol mit Weinsäure, vorzugsweise das Salz von Tapentadol mit L-(+)-Weinsäure, eingebettet ist, wobei die Matrix mit verlängerter Freisetzung als Matrixmaterial mit verlängerter Freisetzung Johannisbrotkernmehl umfasst, vorzugsweise in einer Menge von 5,0 bis 35 Gew.-%, z. B. 15±10 Gew.-% oder 20±10 Gew.-% oder 25±10 Gew.-%, jeweils bezogen auf das Gesamtgewicht der Darreichungsform.

Zusätzlich zu den obigen Inhaltsstoffen kann eine Matrix mit verlängerter Freisetzung auch geeignete Mengen anderer Materialien, z. B. in der Pharmazie übliche Verdünnungsmittel, Schmiermittel, Bindemittel, Granulierhilfsstoffe, Farbstoffe, Geschmacksstoffe und Begleitmittel enthalten.

Pharmazeutische Darreichungsformen, die Matrizen mit verlängerter Freisetzung enthalten, in denen Tapentadol eingebettet ist, lassen sich durch herkömmliche, dem Fachmann bekannte Verfahren wie Mischen und direktes Verpressen, Trockengranulation, Nassgranulation, Extrusion und dergleichen herstellen.

Gemäß einer bevorzugten Ausführungsformen handelt es sich bei der erfindungsgemäßen pharmazeutischen Darreichungsform um eine Kapsel, die vorzugsweise eine Vielzahl von einen Überzug mit verlängerter Freisetzung enthaltenden Partikeln enthält.

Gemäß einer anderen bevorzugten Ausführungsform handelt es sich bei der erfindungsgemäßen pharmazeutischen Darreichungsform um eine Tablette. Die Tablette ist vorzugsweise monolithisch. Erfindungsgemäß sind monolithische Tabletten gegebenenfalls filmbeschichtete Tabletten, bei denen die Tablettenkerne ein komprimiertes Pulver und/oder eine Granulatmischung enthalten. Insbesondere sind (i) durch direktes Verpressen von Pulvermischungen hergestellte Tabletten, (ii) durch Verpressen von durch Trockengranulation erhaltenes Granulat und gegebenenfalls extragranuläre Exzipienten umfassenden Mischungen hergestellte Tabletten, (iii) durch Verpressen von durch Nassgranulation erhaltenes Granulat und gegebenenfalls extragranuläre Exzipienten umfassenden Mischungen hergestellte Tabletten und (iv) durch Verpressen von durch Extrusionsgranulation erhaltenes Granulat und gegebenenfalls extragranuläre Exzipienten umfassenden Mischungen hergestellte Tabletten alle als erfindungsgemäße monolithische Tabletten anzusehen. Multiple Unit Pellet Systems (MUPS) oder andere Darreichungsformen, bei denen eine Vielzahl von Partikeln mit spezifischem Design, spezifischem Gewicht und spezifischer Form mit einem äußeren Matrixmaterial gemischt und anschließend zu Tabletten verpresst werden, wobei das äußere Matrixmaterial eine kontinuierliche Phase bildet, in der die Pellets bzw. Partikel eingebettet sind, werden vorzugsweise nicht als monolithische Tabletten angesehen. Kapseln, die mit einer Vielzahl loser Partikel gefüllt sind, sind natürlich ebenfalls nicht als monolithisch anzusehen.

Die Tablette hat vorzugsweise eine Bruchfestigkeit von mindestens 100 N, vorzugsweise mindestens 150 N, besonders bevorzugt mindestens 200 N. Die Bruchfestigkeit wird vorzugsweise gemäß Ph. Eur. 10, Kapitel 2.9.8. "Resistance to Crushing of Tablets*"* bestimmt.

Ein anderer Aspekt der Erfindung betrifft ein Verfahren zur Herstellung einer wie oben beschriebenen erfindungsgemäßen pharmazeutischen Darreichungsform.

Enthält die pharmazeutische Darreichungsform einen Überzug mit verlängerter Freisetzung umfassende Partikel, so umfasst das erfindungsgemäße Herstellungsverfahren gemäß einer bevorzugten Ausführungsform die folgenden Schritte:
(A) die Bereitstellung innerer Ausgangspellets, z. B. Zuckerkügelchen, die einen oder mehrere Exzipienten, jedoch kein Tapentadol enthalten;
(B) die Bereitstellung einer Lösung oder Dispersion von Tapentadol (einschließlich des Salzes mit Weinsäure, insbesondere L-(+)-Weinsäure) in Wasser oder einem organischen Lösungsmittel oder einer Mischung davon, gegebenenfalls mit dem einen oder den mehreren Exzipienten, wobei das organische Lösungsmittel vorzugsweise aus Ethanol und Aceton ausgewählt ist;
(C) das Beschichten der in Schritt (A) bereitgestellten inneren Ausgangspellets mit der in Schritt (B) bereitgestellten Lösung oder Dispersion von Tapentadol, vorzugsweise in einer Wirbelschicht, unter Erhalt von Zwischenproduktpartikeln, die einen kein Tapentadol enthaltenden inneren Kern und eine den Kern verkapselnde Arzneimittelüberzugsschicht, die im Wesentlichen die gesamte Menge an Tapentadol, die in der Darreichungsform enthalten sein soll, umfasst, gegebenenfalls zusammen mit dem einen oder den mehreren Exzipienten umfassen;
(D) gegebenenfalls das Trocknen der in Schritt (C) erhaltenen Zwischenproduktpartikel unter Erhalt getrockneter Zwischenproduktpartikel;
(E) die Bereitstellung einer Lösung oder Dispersion eines Überzugsmaterials mit verlängerter Freisetzung in Wasser oder eines organischen Lösungsmittels oder einer Mischung davon, gegebenenfalls mit dem einen oder den mehreren Exzipienten, wobei das organische Lösungsmittel vorzugsweise aus Ethanol und Aceton ausgewählt ist;
(F) das Beschichten der in Schritt (C) erhaltenen Zwischenproduktpartikel oder der in Schritt (D) erhaltenen getrockneten Zwischenproduktpartikel mit der in Schritt (E) bereitgestellten Lösung oder Dispersion des Überzugsmaterials mit verlängerter Freisetzung, vorzugsweise in einer Wirbelschicht, unter Erhalt von Partikeln mit verlängerter Freisetzung, die einen kein Tapentadol enthaltenden inneren Kern, eine den Kern verkapselnde Arzneimittelüberzugsschicht, die im Wesentlichen die gesamte Menge an Tapentadol umfasst, gegebenenfalls zusammen mit dem einen oder den mehreren Exzipienten, und einen den Kern und die Arzneimittelüberzugsschicht verkapselnden Überzug mit verlängerter Freisetzung enthalten;
(G) gegebenenfalls das Trocknen der in Schritt (F) erhaltenen Partikel mit verlängerter Freisetzung unter Erhalt getrockneter Partikel mit verlängerter Freisetzung; und
(H) entweder das Abfüllen der in Schritt (F) erhaltenen Partikel mit verlängerter Freisetzung oder der in Schritt (G) erhaltenen getrockneten Partikel mit verlängerter Freisetzung in Kapseln; oder das Mischen der in Schritt (F) erhaltenen Partikel mit verlängerter Freisetzung oder der in Schritt (G) erhaltenen getrockneten Partikel mit verlängerter Freisetzung mit extrapartikulären Exzipienten und das Verpressen der Mischung zu Tabletten (Multiple Unit Pellet Systems, MUPS).

Ein anderer Aspekt der Erfindung betrifft eine durch dieses wie oben beschriebene bevorzugte erfindungsgemäße Verfahren erhältliche pharmazeutische Darreichungsform.

Enthält die pharmazeutische Darreichungsform einen Überzug mit verlängerter Freisetzung umfassende Partikel, so umfasst das erfindungsgemäße Herstellungsverfahren gemäß einer bevorzugten Ausführungsform die folgenden Schritte:
(A) das Bereitstellen einer im Wesentlichen die gesamte Menge an Tapentadol (einschließlich des Salzes mit Weinsäure, insbesondere L-(+)-Weinsäure), die in der Darreichungsform enthalten sein soll, enthaltenden Mischung, gegebenenfalls zusammen mit einem oder mehreren Exzipienten;
(B) die Herstellung von Arzneimittelpellets aus der in Schritt (A) bereitgestellten Mischung durch Trockengranulation, Nassgranulation oder Extrusion, wobei die Nassgranulation vorzugsweise die Verwendung eines aus Wasser, Ethanol, Aceton und einer beliebigen Mischung davon ausgewählten Lösungsmittels umfasst;
(C) gegebenenfalls das Trocknen und/oder Sphäronisieren den Schritt (B) hergestellten Arzneimittelpellets unter Erhalt getrockneter und/oder sphäronisierter Arzneimittelpellets;
(D) die Bereitstellung einer Lösung oder Dispersion eines Überzugsmaterials mit verlängerter Freisetzung in Wasser oder einem organischen Lösungsmittel oder einer Mischung davon, gegebenenfalls mit einem oder mehreren Exzipienten, wobei das organische Lösungsmittel vorzugsweise aus Ethanol und Aceton ausgewählt ist;
(E) das Beschichten der in Schritt (B) hergestellten Arzneimittelpellets oder der in Schritt (C) erhaltenen getrockneten und/oder sphäronisierten Arzneimittelpellets mit der in Schritt (D) bereitgestellten Lösung oder Dispersion des Überzugsmaterials mit verlängerter Freisetzung, vorzugsweise in einer Wirbelschicht, unter Erhalt von Partikeln mit verlängerter Freisetzung, die einen im Wesentlichen die Gesamtmenge an Tapentadol umfassenden Kern, gegebenenfalls zusammen mit einem oder mehreren Exzipienten, und einen den Kern verkapselnden Überzug mit verlängerter Freisetzung enthalten;
(F) gegebenenfalls das Trocknen der in Schritt (E) erhaltenen Partikel mit verlängerter Freisetzung unter Erhalt getrockneter Partikel mit verlängerter Freisetzung; und
(G) entweder das Abfüllen der in Schritt (E) erhaltenen Partikel mit verlängerter Freisetzung oder der in Schritt (F) erhaltenen getrockneten Partikel mit verlängerter Freisetzung in Kapseln; oder das Mischen der in Schritt (E) erhaltenen Partikel mit verlängerter Freisetzung oder den Schritt (F) erhaltenen getrockneten Partikel mit verlängerter Freisetzung mit extrapartikulären Exzipienten und das Verpressen der Mischung zu Tabletten (Multiple Unit Pellet Systems, MUPS).

Ein anderer Aspekt der Erfindung betrifft eine durch dieses wie oben beschriebene bevorzugte erfindungsgemäße Verfahren erhältliche pharmazeutische Darreichungsform.

Enthält die pharmazeutische Darreichungsform einer Matrix mit verlängerter Freisetzung, in der das Tapentadol (einschließlich des Salzes mit Weinsäure, insbesondere L-(+)-Weinsäure) eingebettet ist, so umfasst das erfindungsgemäße Herstellungsverfahren gemäß einer bevorzugten Ausführungsform die folgenden Schritte:
(a) das Bereitstellen einer im Wesentlichen die gesamte Menge an Tapentadol (einschließlich des Salzes mit Weinsäure, insbesondere L-(+)-Weinsäure), die in der Darreichungsform enthalten sein soll, und mindestens ein Matrixmaterial mit verlängerter Freisetzung enthaltenden Mischung, gegebenenfalls zusammen mit einem oder mehreren Exzipienten;
(b) gegebenenfalls das Granulieren der in Schritt (a) bereitgestellten Mischung unter Erhalt eines Granulats, wobei das Granulieren vorzugsweise Folgendes umfasst: (i) Nassgranulation mittels eines vorzugsweise aus Wasser, Ethanol, Aceton und einer beliebigen Mischung davon ausgewählten Lösungsmittels, gegebenenfalls gefolgt von Trocknen; (ii) Trockengranulation; oder (iii) Extrusion;
(c) gegebenenfalls das Mischen des in Schritt (b) erhaltenen Granulats mit einem oder mehreren Exzipienten unter Erhalt einer Granulatmischung;
(d) das Verpressen der in Schritt (a) bereitgestellten Mischung oder des in Schritt (b) erhaltenen Granulats oder der in Schritt (c) erhaltenen Granulatmischung zu Tabletten;
(e) gegebenenfalls das Filmbeschichten der in Schritt (d) verpressten Tabletten.

Das Verpressen in Schritt (d) des erfindungsgemäßen Verfahrens erfolgt vorzugsweise mit einer Presskraft von nicht mehr als 20 kN, besonders bevorzugt nicht mehr als 15 kN, noch mehr bevorzugt nicht mehr als 10 kN, noch mehr bevorzugt nicht mehr als 9,5 kN, noch mehr bevorzugt nicht mehr als 9,0 kN, am meisten bevorzugt nicht mehr als 8,75 kN und insbesondere nicht mehr als 8,5 kN.

Das Verpressen in Schritt (d) des erfindungsgemäßen Verfahrens erfolgt vorzugsweise unter Bedingungen, bei denen die verpresste Tablette eine Bruchfestigkeit von mindestens 100 N, besonders bevorzugt mindestens 150 N, noch mehr bevorzugt mindestens 200 N aufweist.

Die erfindungsgemäße pharmazeutische Darreichungsform wird vorzugsweise nicht durch thermisches Formen wie Schmelzextrusion hergestellt.

Die erfindungsgemäße pharmazeutische Darreichungsform enthält vorzugsweise keine Vielzahl an Partikeln oder Pellets mit spezifischem Design, spezifischer Form und spezifischem Gewicht, die gegebenenfalls zu Tabletten verpresst sind, bei denen die Partikel bzw. Pellets eine diskontinuierliche Phase in einer kontinuierlichen Phase eines äußeren Matrixmaterials bilden.

Die erfindungsgemäße pharmazeutische Darreichungsform enthält vorzugsweise keinen Opioidantagonisten. Opioidantagonisten sind Einheiten, die die Reaktion von Opioidrezeptoren modifizieren. Opioidantagonisten schließen Naloxon, Naltrexon, Diprenorphin, Etorphin, Dihydroetorphin, Nalinefen, Cyclazacin, Levallorphan, pharmazeutisch unbedenkliche Salze davon und Mischungen davon ein.

Ein anderer Aspekt der Erfindung betrifft die wie oben beschriebene erfindungsgemäße pharmazeutische Darreichungsform zur Verwendung bei der Behandlung von Schmerzen, wobei die Darreichungsform oral verabreicht wird, vorzugsweise zweimal täglich.

Ein anderer Aspekt der Erfindung betrifft die Verwendung eines Salzes von Tapentadol mit Weinsäure, von Tapentadol mit L-(+)-Weinsäure zur Herstellung einer wie oben beschriebenen erfindungsgemäßen pharmazeutischen Darreichungsform zur Behandlung von Schmerzen, wobei die Darreichungsform oral verabreicht wird, vorzugsweise zweimal täglich.

Ein anderer Aspekt der Erfindung betrifft ein Verfahren zur Behandlung von Schmerzen, welches den Schritt der oralen, vorzugsweise zweimal täglichen, Verabreichung einer wie oben beschriebenen erfindungsgemäßen pharmazeutischen Darreichungsform an ein dessen bedürftiges Subjekt umfasst.

Bei den Schmerzen handelt es sich vorzugsweise um chronische Schmerzen.

Gemäß bevorzugten Ausführungsformen stellt die erfindungsgemäße pharmazeutische Darreichungsform in einer Patientenpopulation von mindestens 10 Patienten, vorzugsweise mindestens 50 Patienten, nach oraler Verabreichung einen durchschnittlichen Tₘₐₓ-Wert innerhalb des Bereichs von 5,0±3,0 Stunden bereit.

Gemäß bevorzugten Ausführungsformen der erfindungsgemäßen pharmazeutischen Darreichungsform beträgt die gewichtsäquivalente Dosis von in der pharmazeutischen Darreichungsform enthaltenem Tapentadol 50 mg, bezogen auf die freie Base von Tapentadol, wobei die Darreichungsform nach oraler Verabreichung in einer Patientenpopulation von mindestens 50 Patienten einen Durchschnittswert von
- Cₘₐₓ im Bereich von 12±3 ng/ml; und/oder
- AUCₗₐₛₜ im Bereich von 204±50 ng·h/ml; und/oder
- AUC_{∞} im Bereich von 214±50 ng·h/ml bereitstellt.

Gemäß bevorzugten Ausführungsformen der erfindungsgemäßen pharmazeutischen Darreichungsform beträgt die gewichtsäquivalente Dosis von in der pharmazeutischen Darreichungsform enthaltenem Tapentadol 100 mg, bezogen auf die freie Base von Tapentadol, wobei die Darreichungsform nach oraler Verabreichung in einer Patientenpopulation von mindestens 50 Patienten einen Durchschnittswert von
- Cₘₐₓ im Bereich von 29±6 ng/ml; und/oder
- AUCₗₐₛₜ im Bereich von 440±100 ng·h/ml; und/oder
- AUC_{∞} im Bereich von 447±100 ng·h/ml bereitstellt.

Gemäß bevorzugten Ausführungsformen der erfindungsgemäßen pharmazeutischen Darreichungsform beträgt die gewichtsäquivalente Dosis von in der pharmazeutischen Darreichungsform enthaltenem Tapentadol 150 mg, bezogen auf die freie Base von Tapentadol, wobei die Darreichungsform nach oraler Verabreichung in einer Patientenpopulation von mindestens 50 Patienten einen Durchschnittswert von
- Cₘₐₓ im Bereich von 47±9 ng/ml; und/oder
- AUCₗₐₛₜ im Bereich von 662±150 ng·h/ml; und/oder
- AUC_{∞} im Bereich von 665±150 ng·h/ml bereitstellt.

Gemäß bevorzugten Ausführungsformen der erfindungsgemäßen pharmazeutischen Darreichungsform beträgt die gewichtsäquivalente Dosis von in der pharmazeutischen Darreichungsform enthaltenem Tapentadol 200 mg, bezogen auf die freie Base von Tapentadol, wobei die Darreichungsform nach oraler Verabreichung in einer Patientenpopulation von mindestens 50 Patienten einen Durchschnittswert von
- Cₘₐₓ im Bereich von 64±12 ng/ml; und/oder
- AUCₗₐₛₜ im Bereich von 890±200 ng·h/ml; und/oder
- AUC_{∞} im Bereich von 895±200 ng·h/ml bereitstellt.

Gemäß bevorzugten Ausführungsformen der erfindungsgemäßen pharmazeutischen Darreichungsform beträgt die gewichtsäquivalente Dosis von in der pharmazeutischen Darreichungsform enthaltenem Tapentadol 250 mg, bezogen auf die freie Base von Tapentadol, wobei die Darreichungsform nach oraler Verabreichung in einer Patientenpopulation von mindestens 50 Patienten einen Durchschnittswert von
- Cₘₐₓ im Bereich von 85±15 ng/ml; und/oder
- AUCₗₐₛₜ im Bereich von 1.141±250 ng·h/ml; und/oder
- AUC_{∞} im Bereich von 1.145±250 ng·h/ml bereitstellt.

Besonders bevorzugte Ausführungsformen der Erfindung sind im Folgenden als Paragraphen 1 bis 67 zusammengefasst:
Paragraph 1. Eine Tapentadol umfassende pharmazeutische Darreichungsform für die zweimal tägliche Verabreichung;
   wobei Tapentadol als ein Salz mit Weinsäure vorliegt; wobei die Darreichungsform eine verlängerte Freisetzung von Tapentadol bereitstellt; und wobei die in der pharmazeutischen Darreichungsform enthaltene gewichtsäquivalente Dosis von Tapentadol im Bereich von 10 bis 300 mg liegt, bezogen auf die freie Base von Tapentadol.
Paragraph 2. Die pharmazeutische Darreichungsform gemäß Paragraph 1, wobei es sich bei dem Salz um das Salz von Tapentadol mit L-(+)-Weinsäure, ein Solvat, ein Ansolvat und/oder eine polymorphe Form davon handelt.
Paragraph 3. Die pharmazeutische Darreichungsform nach Paragraph 1 oder 2, welche einen, zwei oder mehr physiologisch unbedenkliche Exzipienten umfasst.
Paragraph 4. Die pharmazeutische Darreichungsform nach einem der vorhergehenden Paragraphen, welche nach oraler Verabreichung Plasmaspiegel von Tapentadol bereitstellt, die über eine Dauer von mindestens 6 Stunden für Schmerzlinderung bereitstellen.
Paragraph 5. Die pharmazeutische Darreichungsform nach einem der vorhergehenden Paragraphen, welche ein In-vitro-Auflösungsprofil, gemessen nach der USP-Paddelmethode bei 50 U/min in 900 ml wässrigem Phosphatpuffer bei einem pH-Wert von 6,8 bei 37°C, bereitstellt, bei dem nach 0,5 Stunden 20±20 Gew.-%; vorzugsweise 20±15 Gew.-%; besonders bevorzugt 20±10 Gew.-%; nach 4 Stunden 60±20 Gew.-%; vorzugsweise 60±15 Gew.-%; besonders bevorzugt 60±10 Gew.-%; und nach 12 Stunden mindestens 60 Gew.-%; vorzugsweise mindestens 70 Gew.-%; besonders bevorzugt mindestens 80 Gew.-% des ursprünglich in der Darreichungsform enthaltenen Tapentadol freigesetzt worden sind.
Paragraph 6. Die pharmazeutische Darreichungsform nach einem der vorhergehenden Paragraphen, welche ein In-vitro-Auflösungsprofil, gemessen nach der USP-Paddelmethode bei 50 U/min in 900 ml wässrigem Phosphatpuffer bei einem pH-Wert von 6,8 bei 37°C, bereitstellt, bei dem - nach 1 Stunde 25±15 Gew.-%;- nach 2 Stunden 35±20 Gew.-%;- nach 4 Stunden 50±20 Gew.-%;- nach 8 Stunden 80±20 Gew.-% des ursprünglich in der Darreichungsform enthaltenen Tapentadol freigesetzt worden sind.
Paragraph 7. Die pharmazeutische Darreichungsform nach einem der vorhergehenden Paragraphen, welche ein In-vitro-Auflösungsprofil, gemessen nach der USP-Paddelmethode bei 50 U/min in 900 ml wässrigem Puffer bei einem pH-Wert von 4,5 bei 37°C, bereitstellt, bei dem nach 0,5 Stunden 20±20 Gew.-%; vorzugsweise 20±15 Gew.-%; besonders bevorzugt 20±10 Gew.-%; nach 4 Stunden 60±20 Gew.-%; vorzugsweise 60±15 Gew.-%; besonders bevorzugt 60±10 Gew.-%; und nach 12 Stunden mindestens 60 Gew.-%; vorzugsweise mindestens 70 Gew.-%; besonders bevorzugt mindestens 80 Gew.-%
   des ursprünglich in der Darreichungsform enthaltenen Tapentadol freigesetzt worden sind.
Paragraph 8. Die pharmazeutische Darreichungsform nach einem der vorhergehenden Paragraphen, welche ein In-vitro-Auflösungsprofil, gemessen nach der USP-Paddelmethode bei 50 U/min in 900 ml wässrigem Puffer bei einem pH-Wert von 4,5 bei 37°C, bereitstellt, bei dem - nach 1 Stunde 25±15 Gew.-%;- nach 2 Stunden 35±20 Gew.-%;- nach 4 Stunden 50±20 Gew.-%;- nach 8 Stunden 80±20 Gew.-% des ursprünglich in der Darreichungsform enthaltenen Tapentadol freigesetzt worden sind. Paragraph 9. Die pharmazeutische Darreichungsform nach einem der vorhergehenden Paragraphen, welche ein In-vitro-Auflösungsprofil, gemessen nach der USP-Paddelmethode bei 50 U/min in 900 ml 0,1 N HCl bei einem pH-Wert von 1,0 und 37°C, bereitstellt, bei dem nach 0,5 Stunden 20±20 Gew.-%; vorzugsweise 20±15 Gew.-%; besonders bevorzugt 20±10 Gew.-%; nach 4 Stunden 60±20 Gew.-%; vorzugsweise 60±15 Gew.-%; besonders bevorzugt 60±10 Gew.-%; und nach 12 Stunden mindestens 60 Gew.-%; vorzugsweise mindestens 70 Gew.-%; besonders bevorzugt mindestens 80 Gew.-% des ursprünglich in der Darreichungsform enthaltenen Tapentadol freigesetzt worden sind.
Paragraph 10. Die pharmazeutische Darreichungsform nach einem der vorhergehenden Paragraphen, welche ein In-vitro-Auflösungsprofil, gemessen nach der USP-Paddelmethode bei 50 U/min in 900 ml 0,1 N HCl bei einem pH-Wert von 1,0 und 37°C, bereitstellt, bei dem - nach 1 Stunde 25±10 Gew.-%;-nach 2 Stunden 40±30 Gew.-%;- nach 4 Stunden 60±20 Gew.-%;- nach 8 Stunden 80±20 Gew.-% des ursprünglich in der Darreichungsform enthaltenen Tapentadol freigesetzt worden sind.
Paragraph 11. Die pharmazeutische Darreichungsform nach einem der vorhergehenden Paragraphen, welche Resistenz gegen ethanolinduziertes Dose-Dumping bereitstellt.
Paragraph 12. Die pharmazeutische Darreichungsform nach Paragraph 11, welche eine langsamere In-vitro-Auflösung von Tapentadol in wässrigem ethanolhaltigem Medium als in wässrigem nicht ethanolhaltigem Medium bereitstellt.
Paragraph 13. Die pharmazeutische Darreichungsform nach Paragraph 11 oder 12, welche eine langsamere Auflösung von Tapentadol in 0,1 N HCl mit 40 Vol.-% Ethanol (pH 1,0) als in 0,1 N HCl ohne 40 Vol.-% Ethanol (pH 1,0) bereitstellt, jeweils gemessen nach der USP-Paddelmethode bei 50 U/min in 900 ml bei 37°C.
Paragraph 14. Die pharmazeutische Darreichungsform nach einem der vorhergehenden Paragraphen, bei welcher die gewichtsäquivalente Dosis von in der pharmazeutischen Darreichungsform enthaltenem Tapentadol 25 mg, 50 mg oder 100 mg beträgt, jeweils bezogen auf die freie Base von Tapentadol. Paragraph 15. Die pharmazeutische Darreichungsform nach Paragraph 14, wobei die Darreichungsform ein Gesamtgewicht im Bereich von 150 bis 750 mg hat.
Paragraph 16. Die pharmazeutische Darreichungsform nach einem der Paragraphen 1 bis 13, bei welcher die gewichtsäquivalente Dosis von in der pharmazeutischen Darreichungsform enthaltenem Tapentadol 150 mg, 200 mg oder 250 mg beträgt, jeweils bezogen auf die freie Base von Tapentadol.
Paragraph 17. Die pharmazeutische Darreichungsform nach Paragraph 16, wobei die Darreichungsform ein Gesamtgewicht im Bereich von 300 bis 1200 mg hat.
Paragraph 18. Die pharmazeutische Darreichungsform nach einem der vorhergehenden Paragraphen, wobei die Darreichungsform einen Überzug mit verlängerter Freisetzung, der ein Überzugsmaterial mit verlängerter Freisetzung ausgewählt aus der Gruppe bestehend aus hydrophoben Celluloseethern, Acrylpolymeren, Schellack, Zein, hydrophoben wachsartigen Produkten und Mischungen davon umfasst, enthält.
Paragraph 19. Die pharmazeutische Darreichungsform nach Paragraph 18, wobei das Überzugsmaterial mit verlängerter Freisetzung vorzugsweise aus der aus Acrylsäure- und Methacrylsäure-Copolymeren, Aminoalkylmethacrylat-Copolymeren, Cyanoethylmethacrylaten, Ethoxyethylmethacrylaten, Ethylcellulose, Methacrylsäurealkylamid-Copolymeren, Methacrylsäure-Copolymeren, Methylmethacrylaten, Methylmethacrylat-Copolymeren, Poly(acrylsäure), Poly(methacrylsäure) (anhydrid), Poly(methacrylsäure), Glycidylmethacrylat-Copolymeren, Poly(methylmethacrylat), Poly(methylmethacrylat)-Copolymeren, Polyacrylamid und Polymethacrylat bestehenden Gruppe ausgewählt ist.
Paragraph 20. Die pharmazeutische Darreichungsform nach einem der vorhergehenden Paragraphen, wobei Tapentadol in einer Matrix mit verlängerter Freisetzung eingebettet ist.
Paragraph 21. Die pharmazeutische Darreichungsform nach Paragraph 20, wobei die Matrix mit verlängerter Freisetzung mindestens ein Matrixmaterial mit verlängerter Freisetzung ausgewählt aus der Gruppe bestehend aus hydrophilen oder hydrophoben Polymeren und Kohlenwasserstoffen umfasst oder im Wesentlichen daraus besteht.
Paragraph 22. Die pharmazeutische Darreichungsform nach Paragraph 21, wobei die Matrix mit verlängerter Freisetzung mindestens ein Polymer ausgewählt aus der Gruppe bestehend aus Polysacchariden oder Gummen (z. B. Xanthan, Guaran, Karayagummi, Johannisbrotkernmehl, Natriumalginat, Karobgummi, Chitosan, Polysacchariden von Mannose und Galactose, Pektin, Tragant, Agar); Celluloseethern (z. B. HPMC, HPC, HEC, MC, EC); Celluloseestern (z. B. Celluloseacetat, Celluloseacetatsuccinat, Celluloseacetatphthalat); Polyalkylenglykolen und Polyalkylenoxiden; Polyvinylalkohol (PVA), quervernetztem Polyvinylalkohol (PVA), Polyvinylpyrrolidon (PVP), quervernetztem Polyvinylpyrrolidon (PVP), Polyvinylpyrrolidon-Vinylacetat-Copolymeren, Polyvinylchlorid (PVC), Polyethylenvinylacetat (PVA), Polydimethylsiloxan (PDS), Polyetherurethan (PEU), Polymilchsäure (PLA), Polyglykolsäure (PGA), Polycaprolacton (PCL), Polyanhydriden, Polyorthoestern; Acrylharzen (z.B. quervernetzten Homopolymeren und Copolymeren von Acrylsäuren, Acrylsäure- und Methacrylsäure-Copolymeren, Aminoalkylmethacrylat-Copolymer, Cyanoethylmethacrylat, Ethoxyethylmethacrylaten, Methacrylsäurealkylamid-Copolymer, Methacrylsäure-Copolymeren, Methylmethacrylaten, Methylmethacrylat-Copolymeren, Poly(acrylsäure), Poly(methacrylsäure), Poly(methacrylsäureanhydrid), Glycidylmethacrylat-Copolymeren, Poly(methylmethacrylat), Poly(methylmethacrylat)-Copolymeren, Polyacrylamid, Polyhydroxyethylmethacrylat (PHEMA) und Polymethacrylat) und aus Proteinen gewonnenen Materialien umfasst oder daraus besteht.
Paragraph 23. Die pharmazeutische Darreichungsform nach Paragraph 21 oder 22, wobei die Matrix mit verlängerter Freisetzung mindestens einen Kohlenwasserstoff ausgewählt aus der Gruppe bestehend aus langkettigen (C8-C50, insbesondere C12-C40) Fettsäuren, Fettalkoholen, Glycerylestern von Fettsäuren, Mineralölen, Pflanzenölen und Wachsen umfasst oder im Wesentlichen daraus besteht.
Paragraph 24. Die pharmazeutische Darreichungsform nach einem der Paragraphen 20 bis 23, wobei die Matrix mit verlängerter Freisetzung ein Matrixmaterial mit verlängerter Freisetzung ausgewählt aus der Gruppe bestehend aus (i) Hydroxypropylmethylcellulose (HPMC); (ii) Hydroxypropylcellulose (HPC); (iii) Hydroxyethylcellulose (HEC); (iv) mikrokristalliner Cellulose (MCC); (v) Ethylcellulose (EC); (vi) Polyvinylacetat (PVAc); (vii) Polyvinylpyrrolidon (PVP); (viii) Polyvinylpyrrolidon-Vinylacetat-Copolymer (PVP/PVAc); (ix) Poly(ethylacrylat-co-methylmethacrylat-co-trimethylammonioethyl-methacrylatchlorid); (x) Poly(butylmethacrylat-co-(2-dimethylaminoethyl)methacrylat-co-methylmethacrylat); (xi) Poly(methylmethacrylat-co-methacrylsäure); (xii) Poly(ethylacrylat-co-methacrylsäure); (xiii) Poly(methylacrylat-co-methylmethacrylat-co-methacrylsäure); (xiv) Poly(ethylacrylat-comethylmethacrylat); (xv) Poly(ethylenoxid) (PEO); (xvi) Polyethylenglykol (PEG); (xvii) langkettigem Fettalkohol mit 8 bis 50 Kohlenstoffatomen, vorzugsweise 12 bis 40 Kohlenstoffatomen, der gesättigt oder ungesättigt und geradkettig oder verzweigt sein kann; (xviii) Cetostearylalkohol; (xix) Stearylalkohol; (xx) Cetylalkohol; (xxi) Kohlenwasserstoff ausgewählt aus der Gruppe bestehend aus langkettigen Fettsäuren mit 8 bis 50 Kohlenstoffatomen, vorzugsweise 12 bis 40 Kohlenstoffatomen, der gesättigt oder ungesättigt und geradkettig oder verzweigt sein kann; Glycerylestern solcher langkettigen Fettsäuren, Mineralölen, Pflanzenölen und Wachsen; (xxii) Xanthan; (xxiii) Natriumalginat; (xxiv) Guaran; (xxv) Johannisbrotkernmehl; und beliebigen Mischungen der obigen; wobei der Gehalt an Matrixmaterial mit verlängerter Freisetzung vorzugsweise im Bereich von 15±10 Gew.-% oder 20±10 Gew.-% oder 25±10 Gew.-% oder 30±10 Gew.-% oder 35±10 Gew.-% oder 40±10 Gew.-% oder 45±10 Gew.-% oder 50±10 Gew.-% oder 55±10 Gew.-% oder 60±10 Gew.-% oder 65±10 Gew.-% oder 70±10 Gew.-% oder 75±10 Gew.-% oder 80±10 Gew.-% liegt, umfasst oder im Wesentlichen daraus besteht, jeweils bezogen auf das Gesamtgewicht der Darreichungsform.
Paragraph 25. Die pharmazeutische Darreichungsform nach Paragraph 24, wobei die Matrix mit verlängerter Freisetzung als Matrixmaterial mit verlängerter Freisetzung Hydroxypropylmethylcellulose (HPMC) in einer Menge von 5,0 bis 50 Gew.-%, z. B. 15±10 Gew.-% oder 20±10 Gew.% oder 25±10 Gew.-% oder 30±10 Gew.-% oder 35±10 Gew.-% oder 40±10 Gew.-%, jeweils bezogen auf das Gesamtgewicht der Darreichungsform, umfasst.
Paragraph 26. Die pharmazeutische Darreichungsform nach Paragraph 24, wobei die Matrix mit verlängerter Freisetzung als Matrixmaterial mit verlängerter Freisetzung Hydroxypropylcellulose (HPC) in einer Menge von 10 bis 50 Gew.-%, z. B. 20±10 Gew.-% oder 25±10 Gew.-% oder 30±10 Gew.-% oder 35±10 Gew.-% oder 40±10 Gew.-%, jeweils bezogen auf das Gesamtgewicht der Darreichungsform, umfasst.
Paragraph 27. Die pharmazeutische Darreichungsform nach Paragraph 24, wobei die Matrix mit verlängerter Freisetzung als Matrixmaterial mit verlängerter Freisetzung Hydroxyethylcellulose (HEC) in einer Menge von 5,0 bis 50 Gew.-%, z. B. 15±10 Gew.-% oder 20±10 Gew.-% oder 25±10 Gew.-% oder 30±10 Gew.-% oder 35±10 Gew.-% oder 40±10 Gew.-%, jeweils bezogen auf das Gesamtgewicht der Darreichungsform, umfasst.
Paragraph 28. Die pharmazeutische Darreichungsform nach Paragraph 24, wobei die Matrix mit verlängerter Freisetzung als Matrixmaterial mit verlängerter Freisetzung mikrokristalline Cellulose (MCC) in einer Menge von 10 bis 70 Gew.-%, z. B. 20±10 Gew.-% oder 25±10 Gew.-% oder 30±10 Gew.-% oder 35±10Gew.-% oder 40±10 Gew.-% oder 45±10 Gew.-% oder 50±10 Gew.-% oder 55±10 Gew.-% oder 60±10 Gew.-%, jeweils bezogen auf das Gesamtgewicht der Darreichungsform, umfasst. Paragraph 29. Die pharmazeutische Darreichungsform nach Paragraph 24, wobei die Matrix mit verlängerter Freisetzung als Matrixmaterial mit verlängerter Freisetzung Ethylcellulose (EC) in einer Menge von 5,0 bis 30 Gew.-%, z. B. 15±10 Gew.-% oder 20±10 Gew.-%, jeweils bezogen auf das Gesamtgewicht der Darreichungsform, umfasst.
Paragraph 30. Die pharmazeutische Darreichungsform nach Paragraph 24, wobei die Matrix mit verlängerter Freisetzung als Matrixmaterial mit verlängerter Freisetzung Polyvinylacetat (PVAc) in einer Menge von 25 bis 70 Gew.-%, z. B. 35±10Gew.-% oder 40±10 Gew.-% oder 45±10 Gew.-% oder 50±10 Gew.-% oder 55±10 Gew.-% oder 60±10 Gew.-%, jeweils bezogen auf das Gesamtgewicht der Darreichungsform, umfasst.
Paragraph 31. Die pharmazeutische Darreichungsform nach Paragraph 24, wobei die Matrix mit verlängerter Freisetzung als Matrixmaterial mit verlängerter Freisetzung Polyvinylpyrrolidon (PVP) in einer Menge von 2,0 bis 21 Gew.-%, z. B. 10±5,0 Gew.-% oder 15±5,0 Gew.-%, jeweils bezogen auf das Gesamtgewicht der Darreichungsform, umfasst.
Paragraph 32. Die pharmazeutische Darreichungsform nach Paragraph 24, wobei die Matrix mit verlängerter Freisetzung als Matrixmaterial mit verlängerter Freisetzung Polyvinylpyrrolidon-Vinylacetat-Copolymer (PVP/PVAc) in einer Menge von 1,0 bis 30 Gew.-%, z. B. 15±10 Gew.-% oder 20±10 Gew.-%, jeweils bezogen auf das Gesamtgewicht der Darreichungsform, umfasst.
Paragraph 33. Die pharmazeutische Darreichungsform nach Paragraph 24, wobei die Matrix mit verlängerter Freisetzung als Matrixmaterial mit verlängerter Freisetzung Poly(ethylacrylat-comethylmethacrylat-co-trimethylammonioethylmethacrylatchlorid) in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform, umfasst.
Paragraph 34. Die pharmazeutische Darreichungsform nach Paragraph 24, wobei die Matrix mit verlängerter Freisetzung als Matrixmaterial mit verlängerter Freisetzung Poly(butylmethacrylat-co-(2-dimethylaminoethyl)methacrylat-co-methylmethacrylat) in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform, umfasst.
Paragraph 35. Die pharmazeutische Darreichungsform nach Paragraph 24, wobei die Matrix mit verlängerter Freisetzung als Matrixmaterial mit verlängerter Freisetzung Poly(methylmethacrylat-co-methacrylsäure) in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform, umfasst.
Paragraph 36. Die pharmazeutische Darreichungsform nach Paragraph 24, wobei die Matrix mit verlängerter Freisetzung als Matrixmaterial mit verlängerter Freisetzung Poly(ethylacrylat-co-methacrylsäure) in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform, umfasst.
Paragraph 37. Die pharmazeutische Darreichungsform nach Paragraph 24, wobei die Matrix mit verlängerter Freisetzung als Matrixmaterial mit verlängerter Freisetzung Poly(methylacrylat-co-methylmethacrylat-co-methacrylsäure) in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform, umfasst.
Paragraph 38. Die pharmazeutische Darreichungsform nach Paragraph 24, wobei die Matrix mit verlängerter Freisetzung als Matrixmaterial mit verlängerter Freisetzung Poly(ethylacrylat-co-methylmethacrylat) in einer Menge von 5,0 bis 45 Gew.-%, z. B. 15±10 Gew.-% oder 20±10 Gew.-% oder 25±10 Gew.-% oder 30±10 Gew.-% oder 35±10 Gew.-%, jeweils bezogen auf das Gesamtgewicht der Darreichungsform, umfasst.
Paragraph 39. Die pharmazeutische Darreichungsform nach Paragraph 24, wobei die Matrix mit verlängerter Freisetzung als Matrixmaterial mit verlängerter Freisetzung Poly(ethylenoxid) (PEO) in einer Menge von 25 bis 65 Gew.-%, z. B. 35±10Gew.-% oder 40±10 Gew.-% oder 45±10 Gew.-% oder 50±10 Gew.-% oder 55±10 Gew.-%, jeweils bezogen auf das Gesamtgewicht der Darreichungsform, umfasst.
Paragraph 40. Die pharmazeutische Darreichungsform nach Paragraph 24, wobei die Matrix mit verlängerter Freisetzung als Matrixmaterial mit verlängerter Freisetzung Polyethylenglykol (PEG) in einer Menge von 5,0 bis 35 Gew.-%, z. B. 15±10 Gew.-% oder 20±10 Gew.-% oder 25±10 Gew.-%, jeweils bezogen auf das Gesamtgewicht der Darreichungsform, umfasst.
Paragraph 41. Die pharmazeutische Darreichungsform nach Paragraph 24, wobei die Matrix mit verlängerter Freisetzung als Matrixmaterial mit verlängerter Freisetzung einen langkettigen Fettalkohol mit 8 bis 50 Kohlenstoffatomen, vorzugsweise 12 bis 40 Kohlenstoffatomen, der gesättigt oder ungesättigt und geradkettig oder verzweigt sein kann, in einer Menge von 15 bis 40 Gew.-%, z. B. 25±10 Gew.-% oder 30±10 Gew.-%, jeweils bezogen auf das Gesamtgewicht der Darreichungsform, umfasst. Paragraph 42. Die pharmazeutische Darreichungsform nach Paragraph 24, wobei die Matrix mit verlängerter Freisetzung als Matrixmaterial mit verlängerter Freisetzung Cetostearylalkohol in einer Menge von 15 bis 40 Gew.-%, z. B. 25±10 Gew.-% oder 30±10 Gew.-%, jeweils bezogen auf das Gesamtgewicht der Darreichungsform, umfasst.
Paragraph 43. Die pharmazeutische Darreichungsform nach Paragraph 24, wobei die Matrix mit verlängerter Freisetzung als Matrixmaterial mit verlängerter Freisetzung Stearylalkohol in einer Menge von 15 bis 40 Gew.-%, z. B. 25±10 Gew.-% oder 30±10 Gew.-%, jeweils bezogen auf das Gesamtgewicht der Darreichungsform, umfasst.
Paragraph 44. Die pharmazeutische Darreichungsform nach Paragraph 24, wobei die Matrix mit verlängerter Freisetzung als Matrixmaterial mit verlängerter Freisetzung Cetylalkohol in einer Menge von 15 bis 40 Gew.-%, z. B. 25±10 Gew.-% oder 30±10 Gew.-%, jeweils bezogen auf das Gesamtgewicht der Darreichungsform, umfasst.
Paragraph 45. Die pharmazeutische Darreichungsform nach Paragraph 24, wobei die Matrix mit verlängerter Freisetzung als Matrixmaterial mit verlängerter Freisetzung einen Kohlenwasserstoff ausgewählt aus der Gruppe der langkettigen Fettsäuren mit 8 bis 50 Kohlenstoffatomen, vorzugsweise 12 bis 40 Kohlenstoffatomen, der gesättigt oder ungesättigt und geradkettig oder verzweigt sein kann, Glycerylester solcher langkettigen Fettsäuren, Mineralöle, Pflanzenöle und Wachse umfasst, jeweils in einer Menge von 5,0 bis 70 Gew.-%, z. B. 15±10 Gew.-% oder 20±10 Gew.-% oder 25±10 Gew.-% oder 30±10 Gew.-% oder 35±10Gew.-% oder 40±10 Gew.-% oder 45±10 Gew.-% oder 50±10 Gew.-% oder 55±10 Gew.-% oder 60±10 Gew.-%, jeweils bezogen auf das Gesamtgewicht der Darreichungsform.
Paragraph 46. Die pharmazeutische Darreichungsform nach Paragraph 24, wobei die Matrix mit verlängerter Freisetzung als Matrixmaterial mit verlängerter Freisetzung Xanthan in einer Menge von 15 bis 40 Gew.-%, z. B. 25±10 Gew.-% oder 30±10 Gew.-%, jeweils bezogen auf das Gesamtgewicht der Darreichungsform, umfasst.
Paragraph 47. Die pharmazeutische Darreichungsform nach Paragraph 24, wobei die Matrix mit verlängerter Freisetzung als Matrixmaterial mit verlängerter Freisetzung Natriumalginat in einer Menge von 15 bis 40 Gew.-%, z. B. 25±10 Gew.-% oder 30±10 Gew.-%, jeweils bezogen auf das Gesamtgewicht der Darreichungsform, umfasst.
Paragraph 48. Die pharmazeutische Darreichungsform nach Paragraph 24, wobei die Matrix mit verlängerter Freisetzung als Matrixmaterial mit verlängerter Freisetzung Guaran in einer Menge von 5,0 bis 35 Gew.-%, z. B. 15±10 Gew.-% oder 20±10 Gew.-% oder 25±10 Gew.-%, jeweils bezogen auf das Gesamtgewicht der Darreichungsform, umfasst.
Paragraph 49. Die pharmazeutische Darreichungsform nach Paragraph 24, wobei die Matrix mit verlängerter Freisetzung als Matrixmaterial mit verlängerter Freisetzung Johannisbrotkernmehl in einer Menge von 5,0 bis 35 Gew.-%, z. B. 15±10 Gew.-% oder 20±10 Gew.-% oder 25±10 Gew.-%, jeweils bezogen auf das Gesamtgewicht der Darreichungsform, umfasst.
Paragraph 50. Die pharmazeutische Darreichungsform nach einem der vorhergehenden Paragraphen, bei der es sich um eine Kapsel handelt.
Paragraph 51. Die pharmazeutische Darreichungsform nach einem der Paragraphen 1 bis 49, bei der es sich um eine Tablette handelt.
Paragraph 52. Die pharmazeutische Darreichungsform nach Paragraph 51, wobei die Tablette monolithisch ist.
Paragraph 53. Die pharmazeutische Darreichungsform nach Paragraph 51 oder 52, wobei die Tablette eine Bruchfestigkeit von mindestens 100 N aufweist.
Paragraph 54. Die Darreichungsform nach einem der vorhergehenden Paragraphen zur Verwendung bei der Behandlung von Schmerzen, wobei die Darreichungsform zweimal täglich oral verabreicht wird. Paragraph 55. Die Darreichungsform zur Verwendung nach Paragraph 54, wobei es sich bei den Schmerzen um chronische Schmerzen handelt.
Paragraph 56. Die Darreichungsform zur Verwendung nach Paragraph 54 oder 55, die in einer Patientenpopulation von mindestens 10 Patienten nach oraler Verabreichung einen durchschnittlichen Tₘₐₓ-Wert innerhalb des Bereichs von 5,0±3,0 Stunden bereitstellt.
Paragraph 57. Die Darreichungsform zur Verwendung nach einem der Paragraphen 54 bis 56, wobei die gewichtsäquivalente Dosis von in der pharmazeutischen Darreichungsform enthaltenem Tapentadol 50 mg beträgt, bezogen auf die freie Base von Tapentadol, und wobei die Darreichungsform nach oraler Verabreichung in einer Patientenpopulation von mindestens 10 Patienten einen Durchschnittswert von Cmax im Bereich von 12±3 ng/ml; und/oder AUClast im Bereich von 204±50 ng·h/ml; und/oder AUC∞ im Bereich von 214±50 ng·h/ml bereitstellt.
Paragraph 58. Die Darreichungsform zur Verwendung nach einem der Paragraphen 54 bis 56, wobei die gewichtsäquivalente Dosis von in der pharmazeutischen Darreichungsform enthaltenem Tapentadol 100 mg beträgt, bezogen auf die freie Base von Tapentadol, und wobei die Darreichungsform nach oraler Verabreichung in einer Patientenpopulation von mindestens 10 Patienten einen Durchschnittswert von Cmax im Bereich von 29±6 ng/ml; und/oder AUClast im Bereich von 440±100 ng·h/ml; und/oder AUC_{∞} im Bereich von 447±100 ng·h/ml bereitstellt.
Paragraph 59. Die Darreichungsform zur Verwendung nach einem der Paragraphen 54 bis 56, wobei die gewichtsäquivalente Dosis von in der pharmazeutischen Darreichungsform enthaltenem Tapentadol 150 mg beträgt, bezogen auf die freie Base von Tapentadol, und wobei die Darreichungsform nach oraler Verabreichung in einer Patientenpopulation von mindestens 10 Patienten einen Durchschnittswert von Cmax im Bereich von 47±9 ng/ml; und/oderAUClast im Bereich von 662±150 ng·h/ml; und/oder AUC∞ im Bereich von 665±150 ng·h/ml bereitstellt.
Paragraph 60. Die Darreichungsform zur Verwendung nach einem der Paragraphen 54 bis 56, wobei die gewichtsäquivalente Dosis von in der pharmazeutischen Darreichungsform enthaltenem Tapentadol 200 mg beträgt, bezogen auf die freie Base von Tapentadol, und wobei die Darreichungsform nach oraler Verabreichung in einer Patientenpopulation von mindestens 10 Patienten einen Durchschnittswert von Cmax im Bereich von 64±12 ng/ml; und/oderAUClast im Bereich von 890±200 ng·h/ml; und/oder AUC_{∞} im Bereich von 895±200 ng·h/ml bereitstellt.
Paragraph 61. Die Darreichungsform zur Verwendung nach einem der Paragraphen 54 bis 56, wobei die gewichtsäquivalente Dosis von in der pharmazeutischen Darreichungsform enthaltenem Tapentadol 250 mg beträgt, bezogen auf die freie Base von Tapentadol, und wobei die Darreichungsform nach oraler Verabreichung in einer Patientenpopulation von mindestens 10 Patienten einen Durchschnittswert von Cmax im Bereich von 85±15 ng/ml; und/oderAUClast im Bereich von 1.141±250 ng·h/ml; und/oder AUC_{∞} im Bereich von 1.145±250 ng·h/ml bereitstellt.
Paragraph 62. Ein Verfahren zur Herstellung einer einen Überzug mit verlängerter Freisetzung umfassende Partikel enthaltenden pharmazeutischen Darreichungsform nach einem der vorhergehenden Paragraphen, wobei das Verfahren die folgenden Schritte umfasst: (A) die Bereitstellung innerer Ausgangspellets, die einen oder mehrere Exzipienten, jedoch kein Tapentadol enthalten; (B) die Bereitstellung einer Lösung oder Dispersion von Tapentadol in Wasser oder einem organischen Lösungsmittel oder einer Mischung davon, gegebenenfalls mit dem einen oder den mehreren Exzipienten; (C) das Beschichten der in Schritt (A) bereitgestellten inneren Ausgangspellets mit der in Schritt (B) bereitgestellten Lösung oder Dispersion von Tapentadol, unter Erhalt von Zwischenproduktpartikeln, die einen kein Tapentadol enthaltenden inneren Kern und eine den Kern verkapselnde Arzneimittelüberzugsschicht, die im Wesentlichen die gesamte Menge an Tapentadol, die in der Darreichungsform enthalten sein soll, umfasst, gegebenenfalls zusammen mit dem einen oder den mehreren Exzipienten umfassen; (D) gegebenenfalls das Trocknen der in Schritt (C) erhaltenen Zwischenproduktpartikel unter Erhalt getrockneter Zwischenproduktpartikel; (E) die Bereitstellung einer Lösung oder Dispersion eines Überzugsmaterials mit verlängerter Freisetzung in Wasser oder einem organischen Lösungsmittel oder einer Mischung davon, gegebenenfalls mit dem einem oder den mehreren Exzipienten; (F) das Beschichten der in Schritt (C) erhaltenen Zwischenproduktpartikel oder der in Schritt (D) erhaltenen getrockneten Zwischenproduktpartikel mit der in Schritt (E) bereitgestellten Lösung oder Dispersion des Überzugsmaterials mit verlängerter Freisetzung, unter Erhalt von Partikeln mit verlängerter Freisetzung, die einen kein Tapentadol enthaltenden inneren Kern, eine den Kern verkapselnde Arzneimittelüberzugsschicht, die im Wesentlichen die gesamte Menge an Tapentadol umfasst, gegebenenfalls zusammen mit dem einen oder den mehreren Exzipienten, und einen den Kern und die Arzneimittelüberzugsschicht verkapselnden Überzug mit verlängerter Freisetzung enthalten; (G) gegebenenfalls das Trocknen der in Schritt (F) erhaltenen Partikel mit verlängerter Freisetzung unter Erhalt getrockneter Partikel mit verlängerter Freisetzung; und (H) entweder das Abfüllen der in Schritt (F) erhaltenen Partikel mit verlängerter Freisetzung oder der in Schritt (G) erhaltenen getrockneten Partikel mit verlängerter Freisetzung in Kapseln; oder das Mischen der in Schritt (F) erhaltenen Partikel mit verlängerter Freisetzung oder der in Schritt (G) erhaltenen getrockneten Partikel mit verlängerter Freisetzung mit extrapartikulären Exzipienten und das Verpressen der Mischung zu Tabletten. Paragraph 63. Eine pharmazeutische Darreichungsform, die durch das Verfahren nach Paragraph 62 erhältlich ist.
Paragraph 64. Ein Verfahren zur Herstellung einer einen Überzug mit verlängerter Freisetzung umfassende Partikel enthaltenden pharmazeutischen Darreichungsform nach einem der Paragraphen 1 bis 61, wobei das Verfahren die folgenden Schritte umfasst: (A) das Bereitstellen einer im Wesentlichen die gesamte Menge an Tapentadol, die in der Darreichungsform enthalten sein soll, enthaltenden Mischung, gegebenenfalls zusammen mit einem oder mehreren Exzipienten; (B) die Herstellung von Arzneimittelpellets aus der in Schritt (A) bereitgestellten Mischung durch Trockengranulation, Nassgranulation oder Extrusion; (C) gegebenenfalls das Trocknen und/oder Sphäronisieren der in Schritt (B) hergestellten Arzneimittelpellets unter Erhalt getrockneter und/oder sphäronisierter Arzneimittelpellets; (D) die Bereitstellung einer Lösung oder Dispersion eines Überzugsmaterials mit verlängerter Freisetzung in Wasser oder eines organischen Lösungsmittels oder einer Mischung davon, gegebenenfalls mit einem oder mehreren Exzipienten; (E) das Beschichten der in Schritt (B) hergestellten Arzneimittelpellets oder der in Schritt (C) erhaltenen getrockneten und/oder sphäronisierten Arzneimittelpellets mit der in Schritt (D) bereitgestellten Lösung oder Dispersion des Überzugsmaterials mit verlängerter Freisetzung, unter Erhalt von Partikeln mit verlängerter Freisetzung, die einen im Wesentlichen die Gesamtmenge an Tapentadol umfassenden Kern, gegebenenfalls zusammen mit einem oder mehreren Exzipienten, und einen den Kern verkapselnden Überzug mit verlängerter Freisetzung enthalten; (F) gegebenenfalls das Trocknen der in Schritt (E) erhaltenen Partikel mit verlängerter Freisetzung unter Erhalt getrockneter Partikel mit verlängerter Freisetzung; und (G) entweder das Abfüllen der in Schritt (E) erhaltenen Partikel mit verlängerter Freisetzung oder der in Schritt (F) erhaltenen getrockneten Partikel mit verlängerter Freisetzung in Kapseln; oder das Mischen der in Schritt (E) erhaltenen Partikel mit verlängerter Freisetzung oder der in Schritt (F) erhaltenen getrockneten Partikel mit verlängerter Freisetzung mit extrapartikulären Exzipienten und das Verpressen der Mischung zu Tabletten.
Paragraph 65. Eine pharmazeutische Darreichungsform, die durch das Verfahren nach Paragraph 64 erhältlich ist.
Paragraph 66. Ein Verfahren zur Herstellung einer eine Matrix mit verlängerter Freisetzung, in der Tapentadol eingebettet ist, enthaltenden pharmazeutischen Darreichungsform nach einem der Paragraphen 1 bis 61, wobei das Verfahren die folgenden Schritte umfasst: (a) das Bereitstellen einer im Wesentlichen die gesamte Menge an Tapentadol, die in der Darreichungsform enthalten sein soll, und mindestens ein Matrixmaterial mit verlängerter Freisetzung enthaltenden Mischung, gegebenenfalls zusammen mit einem oder mehreren Exzipienten; (b) gegebenenfalls das Granulieren der in Schritt (a) bereitgestellten Mischung unter Erhalt eines Granulats, (c) gegebenenfalls das Mischen des in Schritt (b) erhaltenen Granulats mit einem oder mehreren Exzipienten unter Erhalt einer Granulatmischung; (d) das Verpressen der in Schritt (a) bereitgestellten Mischung oder des in Schritt (b) erhaltenen Granulats oder der in Schritt (c) erhaltenen Granulatmischung zu Tabletten; (e) gegebenenfalls das Filmbeschichten der in Schritt (d) verpressten Tabletten.
Paragraph 67. Das Verfahren nach Paragraph 66, wobei das Verpressen in Schritt (d) mit einer Presskraft von nicht mehr als 20 kN, besonders bevorzugt nicht mehr als 15 kN, noch mehr bevorzugt nicht mehr als 10 kN, noch mehr bevorzugt nicht mehr als 9,5 kN, noch mehr bevorzugt nicht mehr als 9,0 kN, am meisten bevorzugt nicht mehr als 8,75 kN und insbesondere nicht mehr als 8,5 kN erfolgt.

### BEISPIELE

Die folgenden Beispiele erläutern die Erfindung näher, sollten jedoch nicht als deren Umfang einschränkend ausgelegt werden.

Im Handel erhältliche Tapentadol-Tabletten Palexia^{®} retard enthalten Tapentadol als Hydrochloridsalz, wobei der Tablettenkern zusätzlich Hypromellose, mikrokristalline Cellulose, feinteiliges Siliciumdioxid und Magnesiumstearat enthält. Palexia^{®} retard-Tabletten enthalten somit Hypromellose als Matrix mit verlängerter Freisetzung. Diese Tabletten entsprechen WO 03/035053 A1 und den im Folgenden beschriebenen Vergleichsbeispielen.

### Beispiel 1 - Herstellung des Salzes von Tapentadol mit L-(+)-Weinsäure

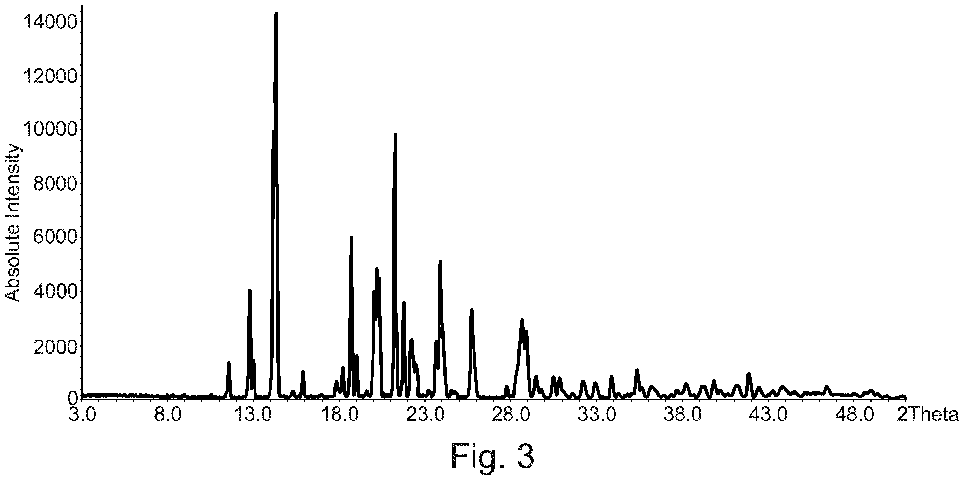

30 g Tapentadol Base wurden mit 20.4 g L-(+)-Weinsäure in einem 1L Reaktor vorgelegt. 400 ml Aceton wurden hinzugegeben und der Ansatz wurde unter starkem Rühren auf 50°C erwärmt und für 3h gerührt. Dabei bildete sich eine klebrige Paste. Danach wurde die Mischung auf 25°C abgekühlt und 200 ml Cyclohexan hinzugegeben. Es wurde auf 5°C gekühlt und bei dieser Temperatur für eine weiter Stunde gerührt. Der Ansatz wurde erneut auf 50°C erwärmt, für 1h gerührt und danach auf 5°C gekühlt. Der Ansatz rührte bei 5°C für 16h nach. Der erhaltene Feststoff wurde abfiltriert und bei 50°C unter reduziertem Druck für 6h getrocknet. 48.1 g Tapentadoltartrat wurden erhalten (Ausbeute 95.5%, 1:1 Stöchiometrie bestimmt per ¹H-NMR, XRPD gemäß Figur 3, DSC: Tₒₙₛₑₜ 131.4°C, Tₚₑₐₖ 134.2°C, TGA: 0% Massenverlust (37°C-172°C)).

### Beispiel 2 - intrinsische Auflösung (Auflösungsrate):

100 mg Tapentadol, entweder in Form des Hydrochloridsalzes oder des Salzes mit L-(+)-Weinsäure wurden mit einer Presskraft von 200 kg (gravitativ) bei einer Oberfläche von 0,5 cm² 1 Minute verpresst. Die so erhaltenen verpressten Proben wurden in einem Wood-Apparat bei 37°C in 900 ml Auflösungsmedium bei verschiedenen pH-Werten und einer Paddeldrehgeschwindigkeit von 50 U/min auf ihre Auflösungsrate untersucht.

Die experimentellen Ergebnisse für Tapentadolhydrochlorid und für das Salz mit L-(+)-Weinsäure sind in der untenstehenden Tabelle zusammengestellt:

| [%] | Tapentadolhydrochlorid (Vergleich) | | | | | Salz von Tapentadol mit L-(+)-Weinsäure (germäß Beispiel 1) | | | |
|---|---|---|---|---|---|---|---|---|---|
| min | pH 1,0 | pH 4,5 | pH 7,4 | pH 6,8 | | pH 1,0 | pH 4,5 | pH 7,4 | pH 6,8 |
| 0 | 0 | 0 | 0 | 0 | | 0 | 0 | 0 | 0 |
| 1 | 52 | 60 | 27 | 38 | | 19 | 20 | 18 | 19 |
| 3 | 95 | 91 | 65 | 79 | | 51 | 51 | 44 | 44 |
| 5 | 99 | 95 | 80 | 92 | | 77 | 77 | 65 | 67 |

### Beispiel 3 - Thermodynamische Löslichkeit:

Die thermodynamische Löslichkeit von Tapentadolhydrochlorid und des Salzes von Tapentadol mit L-(+)-Weinsäure wurde als Sättigungslöslichkeit in verschiedenen Medien bei verschiedenen pH-Werten bestimmt. Die Lösungen wurden 24 Stunden bei 25°C gerührt und die pH-Werte der Lösungen zu Beginn und am Ende der Experimente wurden gemessen. Die gelöste Menge an Tapentadol wurde durch HPLC quantifiziert (freie Base von Tapentadol). Die experimentellen Ergebnisse sind in der Tabelle unten zusammengestellt:

| | Tapentadolhydrochlorid (Vergleich) | | | | Salz von Tapentadol mit L-(+)-Weinsäure (gemäß Beispiel 1) | | |
|---|---|---|---|---|---|---|---|
| | pH | | Assay ausgedrückt als Base | | pH | | Assay ausgedrückt als Base |
| | Start | Ende | g/100 ml | | Start | Ende | g/100 ml |
| 0,1 M HCl | 1,11 | 0,83 | 31,2 | | 1,15 | 3,39 | 26,7 |
| Acetat pH 4,5 | 4,54 | 4,29 | 32,7 | | 4,51 | 3,53 | 26,6 |
| Wasser | n.b. | 4,63 | 33,0 | | 6,19 | 3,38 | 26,4 |
| SIF sp pH 6,8 | 6,82 | 6,23 | 32,4 | | 6,83 | 3,55 | 12,6 |
| Phosphat pH 7,4 | n.b. | n.b. | n.b. | | 7,53 | 3,56 | 12,9 |
| Citrat pH 7.4 | 7,39 | 6,33 | 31,9 | | n.b. | n.b. | n.b. |

| | | | | | | | |
|---|---|---|---|---|---|---|---|
| n.b. = nicht bestimmt | | | | | | | |

### Beispiel 4 - In-vitro-Auflösungsprofile von pharmazeutischen Darreichungsformen:

Tabletten mit der folgenden Zusammensetzung wurden durch Mischen aller Bestandteile und Verpressen der erhaltenen Mischungen hergestellt:

| | Vergleich | erfindungsgemäß |
|---|---|---|
| | C-1 | I-1 |
| [mg] | | |
| Tapentadolhydrochlorid¹ | 250,00 | - |
| Salz von Tapentadol mit L-(+)-Weinsäure^{1,2} | - | 250,00 |
| Hydroxypropylmethylcellulose 100000 mPa·s Typ 2208 | 100,00 | 100,00 |
| silifizierte mikrokristalline Cellulose | 304,80 | 304,80 |

| (Prosolv HD90) | | |
|---|---|---|
| Magnesiumstearat | 4,00 | 4,00 |
| mittlere Bruchfestigkeit, Ph. Eur. 2.9.8. [N] | 222 | 239 |
| mittlere Presskraft, oberer Stempel [kN] | 12,8 | 8,0 |
| mittlere Presskraft, unterer Stempel [kN] | 11,3 | 7,0 |

| | | |
|---|---|---|
| ¹ äquivalente Dosis bezogen auf die freie Base von Tapentadol; ² gemäß Beispiel 1 | | |

Aus den obigen Vergleichsdaten ist ersichtlich, dass man für Tabletten der gleichen Dosisstärke an Tapentadol zum Erhalt von Tabletten mit verbesserter Bruchfestigkeit eine signifikant niedrigere Presskraft benötigt, wenn man Tapentadol als Salz mit L-(+)-Weinsäure anstelle von Salzsäure einsetzt, (siehe z. B. C-1 vs. 1-1). Die Tabletten C-1 und I-1 beispielsweise haben eine mittlere Bruchfestigkeit von 222 bzw. 239 N; das Hydrochloridsalz erfordert Tablettierkräfte von 12,8 kN beziehungsweise 11,3 kN, während das L-(+)-Weinsäuresalz nur 8,0 kN beziehungsweise 7,0 kN benötigt.

Die In-vitro-Auflösung von Tapentadol aus den Tabletten wurde in einem mit Sinker ausgestatteten Paddelapparat gemäß Ph. Eur. bei einer Umdrehungsgeschwindigkeit von 50 U/min bei 37°C in 900 ml verschiedener Auflösungsmedien mit verschiedenen pH-Werten gemessen. Die experimentellen Ergebnisse sind in den nachstehenden Tabellen zusammengestellt:

| [%] | Tapentadolhydrochlorid (n=3): | | | | |
|---|---|---|---|---|---|
| | C-1 | | | | |
| Zeit [min] | pH 6,8 | pH 4,5 | pH 1,0 | pH 1,0 40 Vol.-% Ethanol | Differenz zwischen pH 1,0 und pH 1,0 40 Vol.-% Ethanol |
| 0 | 0 | 0 | 0 | 0 | 0 |
| 30 | 17 | 19 | 18 | 14 | 4 |
| 60 | 26 | 28 | 27 | 22 | 5 |
| 90 | 33 | 36 | 34 | 28 | 6 |
| 120 | 39 | 42 | 40 | 33 | 7 |
| 240 | 58 | 63 | 60 | 50 | 10 |
| 480 | 82 | 87 | 84 | 73 | 11 |
| 720 | 93 | 96 | 95 | 87 | 8 |

| [%] | Salz von Tapentadol mit L-(+)-Weinsäure (n=3): | | | | |
|---|---|---|---|---|---|
| | I-1 | | | | |
| Zeit [min] | pH 6,8 | pH 4,5 | pH 1,0 | pH 1,0 40 Vol.-% Ethanol | Differenz zwischen pH 1,0 und pH 1,0 40 Vol.-% Ethanol |
| 0 | 0 | 0 | 0 | 0 | 0 |
| 30 | 15 | 14 | 17 | 11 | 6 |
| 60 | 24 | 23 | 28 | 20 | 8 |
| 90 | 31 | 30 | 36 | 26 | 10 |
| 120 | 37 | 36 | 43 | 32 | 11 |
| 240 | 57 | 56 | 64 | 50 | 14 |
| 480 | 83 | 82 | 90 | 75 | 15 |
| 720 | 96 | 95 | 99 | 91 | 8 |

### Beispiel 5 - In-vitro-Auflösungsprofile von pharmazeutischen Darreichungsformen:

Tabletten mit der folgenden Zusammensetzung wurden durch Mischen aller Bestandteile und Verpressen der erhaltenen Mischungen hergestellt:

| | Vergleich | erfindungsgemäß |
|---|---|---|
| [mg] | C-2 | I-2 |
| Tapentadolhydrochlorid¹ | 250,00 | - |
| Salz von Tapentadol mit L-(+)-Weinsäure^{1,2} | - | 250,00 |
| Kollidon^{®} SR | 320,00 | 320,00 |
| Aerosil 200 | 6,80 | 6,80 |
| Magnesiumstearat | 3,20 | 3,20 |
| mittlere Bruchfestigkeit, Ph. Eur. 2.9.8. [N] | 237 | 271 |
| mittlere Presskraft, oberer Stempel [kN] | 12,4 | 8,1 |
| mittlere Presskraft, unterer Stempel [kN] | 9,9 | 6,5 |

| | | |
|---|---|---|
| ¹ äquivalente Dosis bezogen auf die freie Base von Tapentadol; ² gemäß Beispiel 1 | | |

Die In-vitro-Auflösung von Tapentadol aus den Tabletten wurde in einem mit Sinker ausgestatteten Paddelapparat gemäß Ph. Eur. bei einer Umdrehungsgeschwindigkeit von 50 U/min bei 37°C in 900 ml 0,1 N HCl ohne Ethanol (pH 1,0) und in 0,1 N HCl mit 40 Vol.-% Ethanol (pH 1,0) gemessen. Die experimentellen Ergebnisse sind in der Tabelle unten zusammengestellt:

| [%] | Tapentadolhydrochlorid (n=3): | | | Salz von Tapentadol mit L-(+)-Weinsäure (n=3): | | |
|---|---|---|---|---|---|---|
| | C-2 | | | I-2 | | |
| Zeit [min] | pH 1,0 | pH 1,0 40 Vol.-% Ethanol | Differenz zwischen pH 1,0 und pH 1,0 40 Vol.-% Ethanol | pH 1,0 | pH 1,0 40 Vol.-% Ethanol | Differenz zwischen pH 1,0 und pH 1,0 40 Vol.-% Ethanol |
| 0 | 0 | 0 | 0 | 0 | 0 | 0 |
| 30 | 24 | 14 | 10 | 33 | 15 | 18 |
| 60 | 33 | 21 | 12 | 46 | 24 | 22 |
| 90 | 40 | 27 | 13 | 55 | 31 | 24 |
| 120 | 45 | 32 | 13 | 63 | 37 | 26 |
| 240 | 60 | 51 | 9 | 84 | 61 | 23 |
| 480 | 79 | 78 | 1 | 100 | 95 | 5 |
| 720 | 90 | 92 | -2 | 103 | 102 | 1 |

Aus den obigen Vergleichsdaten ist ersichtlich, dass die reduzierte Presskraft des L-(+)-Weinsäuresalzes von Tapentadol im Vergleich zum Hydrochloridsalz von Tapentadol nicht von den anderen Exzipienten der Tablette abhängt (C-1, I-1 beide Hydroxypropylmethylcellulose), sondern auch mit Kollidon^{®} SR beobachtet wird. Wiederum ist für Tabletten der gleichen Dosisstärke an Tapentadol eine signifikant niedrigere Presskraft erforderlich, wenn man Tapentadol als Salz mit L-(+)-Weinsäure anstelle von Salzsäure zur Bereitstellung von Tabletten mit verbesserter Bruchfestigkeit einsetzt (siehe C-2 vs. 1-2). Die Tabletten C-2 und I-2 haben beide eine mittlere Bruchfestigkeit von etwa 237 bzw. 271 N; das Hydrochloridsalz erfordert Tablettierkräfte von 12,4 kN beziehungsweise 9,9 kN, während das Salz der L-(+)-Weinsäure nur 8,1 kN beziehungsweise 6,5 kN benötigt.

## Patentansprüche

1. Tapentadol umfassende pharmazeutische Darreichungsform für die zweimal tägliche Verabreichung;
wobei Tapentadol als Salz mit Weinsäure vorliegt;
wobei die Gewichtsäquivalentdosis von Tapentadol, die in der pharmazeutischen Darreichungsform enthalten ist, im Bereich von 10 bis 300 mg, bezogen auf die freie Base von Tapentadol, liegt;
wobei die Darreichungsform eine Tablette ist;
wobei die Darreichungsform eine verlängerte Freisetzung von Tapentadol bereitstellt; und
wobei Tapentadol in einer Matrix mit verlängerter Freisetzung eingebettet ist.

2. Pharmazeutische Darreichungsform nach Anspruch 1, wobei es sich bei dem Salz um das Salz von Tapentadol mit L-(+)-Weinsäure, ein Solvat, ein Ansolvat und/oder eine polymorphe Form davon, eine kristalline Form und/oder eine amorphe Form davon handelt.

3. Pharmazeutische Darreichungsform nach Anspruch 1 oder 2, wobei die Tablette monolithisch ist.

4. Die pharmazeutische Darreichungsform nach einem der vorhergehenden Ansprüche, wobei die Tablette eine Bruchfestigkeit von mindestens 100 N aufweist, bestimmt gemäß Ph. Eur. 2.9.8.

5. Pharmazeutische Darreichungsform nach einem der vorhergehenden Ansprüche, wobei die gewichtsäquivalente Dosis von in der pharmazeutischen Darreichungsform enthaltenem Tapentadol 25 mg, 50 mg oder 100 mg, jeweils bezogen auf die freie Base von Tapentadol beträgt; und wobei die Darreichungsform ein Gesamtgewicht im Bereich von 150 bis 750 mg hat.

6. Pharmazeutische Darreichungsform nach einem der Ansprüche 1 bis 4, wobei die gewichtsäquivalente Dosis von in der pharmazeutischen Darreichungsform enthaltenem Tapentadol 150 mg, 200 mg oder 250 mg, jeweils bezogen auf die freie Base von Tapentadol beträgt; und wobei die Darreichungsform ein Gesamtgewicht im Bereich von 300 bis 1.200 mg hat.

7. Pharmazeutische Darreichungsform nach einem der vorhergehenden Ansprüche, wobei die Matrix mit verlängerter Freisetzung ein Matrixmaterial mit verlängerter Freisetzung ausgewählt aus der Gruppe bestehend aus
(i) Hydroxypropylmethylcellulose (HPMC);
(ii) Hydroxypropylcellulose (HPC);
(iii) Hydroxyethylcellulose (HEC);
(iv) mikrokristalliner Cellulose (MCC);
(v) Ethylcellulose (EC);
(vi) Polyvinylacetat (PVAc);
(vii) Polyvinylpyrrolidon (PVP);
(viii) Polyvinylpyrrolidon-Vinylacetat-Copolymer (PVP/PVAc);
(ix) Poly(ethylacrylat-co-methylmethacrylat-co-trimethylammonioethylmethacrylatchlorid);
(x) Poly(butylmethacrylat-co-(2-dimethylaminoethyl)methacrylat-co-methylmethacrylat);
(xi) Poly(methylmethacrylat-co-methacrylsäure);
(xii) Poly(ethylacrylat-co-methacrylsäure);
(xiii) Poly(methylacrylat-co-methylmethacrylat-co-methacrylsäure);
(xiv) Poly(ethylacrylat-co-methylmethacrylat);
(xv) Poly(ethylenoxid) (PEO);
(xvi) Polyethylenglykol (PEG);
(xvii) langkettigem Fettalkohol mit 8 bis 50 Kohlenstoffatomen, vorzugsweise 12 bis 40 Kohlenstoffatomen, der gesättigt oder ungesättigt und geradkettig oder verzweigt sein kann;
(xviii) Cetostearylalkohol;
(xix) Stearylalkohol;
(xx) Cetylalkohol;
(xxi) Kohlenwasserstoff ausgewählt aus der Gruppe bestehend aus langkettigen Fettsäuren mit 8 bis 50 Kohlenstoffatomen, vorzugsweise 12 bis 40 Kohlenstoffatomen, die gesättigt oder ungesättigt und geradkettig oder verzweigt sein können; Glycerylestern solcher langkettigen Fettsäuren, Mineralölen, Pflanzenölen und Wachsen;
(xxii) Xanthan;
(xxiii) Natriumalginat;
(xxiv) Guaran;
(xxv) Johannisbrotkernmehl; und
beliebigen Mischungen der obigen umfasst oder im Wesentlichen daraus besteht.

8. Pharmazeutische Darreichungsform nach Anspruch 7, wobei der Gehalt an Matrixmaterial mit verlängerter Freisetzung vorzugsweise im Bereich von 15±10 Gew.-% oder 20±10 Gew.-% oder 25±10 Gew.-% oder 30±10 Gew.-% oder 35±10 Gew.-% oder 40±10 Gew.-% oder 45±10 Gew.-% oder 50±10 Gew.-% oder 55±10 Gew.-% oder 60±10 Gew.-% oder 65±10 Gew.-% oder 70±10 Gew.-% oder 75±10 Gew.-% oder 80±10 Gew.-%, jeweils bezogen auf das Gesamtgewicht der Darreichungsform, liegt.

9. Pharmazeutische Darreichungsform nach einem der vorhergehenden Ansprüche zur Verwendung bei der Behandlung von Schmerzen.

10. Pharmazeutische Darreichungsform zur Verwendung nach Anspruch 9, wobei die Darreichungsform oral verabreicht wird.

11. Pharmazeutische Darreichungsform zur Verwendung nach Anspruch 9 oder 10, wobei die Darreichungsform zweimal täglich verabreicht wird.

12. Pharmazeutische Darreichungsform zur Verwendung nach einem der Ansprüche 9 bis 11, welche nach oraler Verabreichung Plasmaspiegel von Tapentadol bereitstellt, die über eine Dauer von mindestens 6 Stunden Schmerzlinderung bereitstellen.

13. Pharmazeutische Darreichungsform zur Verwendung nach einem der Ansprüche 9 bis 12, wobei es sich bei den Schmerzen um chronische Schmerzen handelt.

14. Verfahren zur Herstellung einer pharmazeutischen Darreichungsform nach einem der Ansprüche 1 bis 13, wobei das Verfahren die folgenden Schritte umfasst:
(a) das Bereitstellen einer im Wesentlichen die gesamte Menge an Tapentadol, die in der Darreichungsform enthalten sein soll, und mindestens ein Matrixmaterial mit verlängerter Freisetzung enthaltenden Mischung, gegebenenfalls zusammen mit einem oder mehreren Exzipienten;
(b) gegebenenfalls das Granulieren der in Schritt (a) bereitgestellten Mischung unter Erhalt eines Granulats:
(c) gegebenenfalls das Mischen des in Schritt (b) erhaltenen Granulats mit einem oder mehreren Exzipienten unter Erhalt einer Granulatmischung;
(d) das Verpressen der in Schritt (a) bereitgestellten Mischung oder des in Schritt (b) erhaltenen Granulats oder der in Schritt (c) erhaltenen Granulatmischung zu Tabletten;
(e) gegebenenfalls das Filmbeschichten der in Schritt (d) verpressten Tabletten.

15. Verfahren nach Anspruch 14, wobei das Verpressen in Schritt (d) mit einer Presskraft von nicht mehr als 20 kN, besonders bevorzugt nicht mehr als 15 kN, noch mehr bevorzugt nicht mehr als 10 kN, noch mehr bevorzugt nicht mehr als 9,5 kN, noch mehr bevorzugt nicht mehr als 9,0 kN, am meisten bevorzugt nicht mehr als 8,75 kN und insbesondere nicht mehr als 8,5 kN erfolgt.

## Claims

1. A pharmaceutical dosage form comprising Tapentadol for twice daily administration;
wherein Tapentadol is present as a salt with tartaric acid;
wherein the weight equivalent dose of Tapentadol that is contained in the pharmaceutical dosage form is in the range of 10 to 300 mg relative to the free base of Tapentadol;
wherein the dosage form is a tablet;
wherein the dosage form provides a prolonged release of Tapentadol; and
wherein Tapentadol is embedded in a prolonged-release matrix.

2. The pharmaceutical dosage form according to claim 1, wherein the salt is the salt of Tapentadol with L-(+)-tartaric acid, a solvate, an ansolvate and/or a polymorphic form thereof, a crystalline form and/or an amorphic form thereof.

3. The pharmaceutical dosage form according to claim 1 or 2, wherein the tablet is monolithic.

4. The pharmaceutical dosage form according to any one of the preceding claims, wherein the tablet has a breaking strength of at least 100 N, determined according to Ph. Eur. 2.9.8.

5. The pharmaceutical dosage form according to any one of the preceding claims, wherein the weight equivalent dose of Tapentadol contained in the pharmaceutical dosage form is 25 mg, 50 mg or 100 mg, in each case relative to the free base of Tapentadol; and wherein the dosage form has a total weight in the range of 150 to 750 mg.

6. The pharmaceutical dosage form according to any one of claims 1 to 4, wherein the weight equivalent dose of Tapentadol contained in the pharmaceutical dosage form is 150 mg, 200 mg or 250 mg, in each case relative to the free base of Tapentadol; and wherein the dosage form has a total weight in the range of 300 to 1200 mg.

7. The pharmaceutical dosage form according to any one of the preceding claims, wherein the prolonged-release matrix comprises or substantially consists of a prolonged-release matrix material selected from the group consisting of
(i) hydroxypropylmethylcellulose (HPMC);
(ii) hydroxypropylcellulose (HPC);
(iii) hydroxyethylcellulose (HEC);
(iv) microcrystalline cellulose (MCC);
(v) ethylcellulose (EC);
(vi) polyvinyl acetate (PVAc);
(vii) polyvinylpyrrolidone (PVP);
(viii) polyvinylpyrrolidone-vinylacetate copolymer (PVP/PVAc);
(ix) poly(ethyl acrylate-co-methyl methacrylate-co-trimethylammonioethyl methacrylate chloride);
(x) poly(butyl methacrylate-co-(2-dimethylaminoethyl) methacrylate-co-methyl methacrylate);
(xi) poly(methyl methacrylate-co-methacrylic acid);
(xii) poly(ethyl acrylate-co-methacrylic acid);
(xiii) poly(methyl acrylate-co-methyl methacrylate-co-methacrylic acid);
(xiv) poly(ethyl acrylate-co-methyl methacrylate);
(xv) poly(ethylene oxide) (PEO);
(xvi) polyethylene glycol (PEG);
(xvii) long chain fatty alcohol having 8 to 50 carbon atoms, preferably 12 to 40 carbon atoms, which may be saturated or unsaturated and linear or branched;
(xviii) cetostearyl alcohol;
(xix) stearyl alcohol;
(xx) cetyl alcohol;
(xxi) hydrocarbon selected from the group consisting of long chain fatty acids having 8 to 50 carbon atoms, preferably 12 to 40 carbon atoms, which may be saturated or unsaturated and linear or branched; glyceryl esters of such long chain fatty acids, mineral oils, vegetable oils, and waxes;
(xxii) xanthan gum;
(xxiii) sodium alginate;
(xxiv) guar gum;
(xxv) locust bean gum; and
any mixtures of the above.

8. The pharmaceutical dosage form according to claim 7, wherein the content of prolonged-release matrix material is preferably in the range of 15±10 wt.-%, or 20±10 wt.-%, or 25±10 wt.-%, or 30±10 wt.-%, or 35±10 wt.-%, or 40±10 wt.-%, or 45±10 wt.-%, or 50±10 wt.-%, or 55±10 wt.-%, or 60±10 wt.-%, or 65±10 wt.-%, or 70±10 wt.-%, or 75±10 wt.-%, or 80±10 wt.-%, in each case relative to the total weight of the dosage form.

9. The pharmaceutical dosage form according to any one of the preceding claims for use in the treatment of pain.

10. The pharmaceutical dosage form for use according to claim 9, wherein the dosage form is orally administered.

11. The pharmaceutical dosage form for use according to claim 9 or 10, wherein the dosage form is administered twice daily.

12. The pharmaceutical dosage form for use according to any one of claims 9 to 11, which, after oral administration, provides plasma levels of Tapentadol providing pain relief for a duration of at least 6 hours.

13. The pharmaceutical dosage form for use according to any one of claims 9 to 12, wherein the pain is chronic pain.

14. A method for the preparation of a pharmaceutical dosage form according to any one of claims 1 to 13, wherein the method comprises the following steps:
(a) providing a mixture containing substantially the entire amount of Tapentadol to be contained in the dosage form and at least one prolonged-release matrix material, optionally together with one or more excipients;
(b) optionally granulating the mixture provided in step (a), thereby obtaining a granulate;
(c) optionally mixing the granulate obtained in step (b) with one or more excipients, thereby obtaining a granulate mixture;
(d) compressing the mixture provided in step (a) or the granulate obtained in step (b) or the granulate mixture obtained in step (c) into tablets;
(e) optionally, film coating the tablets compressed in step (d).

15. The method according to claim 14, wherein the compression in step (d) occurs with a compression force of not more than 20 kN, particularly preferably not more than 15 kN, even more preferably not more than 10 kN, even more preferably not more than 9.5 kN, even more preferably not more than 9.0 kN, most preferably not more than 8.75 kN and in particular not more than 8.5 kN.

## Revendications

1. Forme posologique pharmaceutique comprenant du Tapentadol pour une administration biquotidienne ;
dans laquelle le Tapentadol est présent sous la forme d'un sel avec de l'acide tartrique ;
dans laquelle la dose équivalente en poids de Tapentadol qui est contenue dans la forme posologique pharmaceutique est dans la plage de 10 à 300 mg par rapport à la base libre de Tapentadol ;
dans laquelle la forme posologique est un comprimé ;
dans laquelle la forme posologique fournit une libération prolongée de Tapentadol ; et
dans laquelle le Tapentadol est incorporé dans une matrice à libération prolongée.

2. Forme posologique pharmaceutique selon la revendication 1, dans laquelle le sel est le sel de Tapentadol avec l'acide L-(+)-tartrique, un solvate, un ansolvate et/ou une forme polymorphe de celui-ci, une forme cristalline et/ou une forme amorphe de celui-ci.

3. Forme posologique pharmaceutique selon la revendication 1 ou 2, dans laquelle le comprimé est monolithique.

4. Forme posologique pharmaceutique selon l'une quelconque des revendications précédentes, dans laquelle le comprimé a une résistance à la rupture d'au moins 100 N, déterminée selon le Ph. Eur. 2.9.8.

5. Forme posologique pharmaceutique selon l'une quelconque des revendications précédentes, dans laquelle la dose équivalente en poids de Tapentadol contenue dans la forme posologique pharmaceutique est de 25 mg, 50 mg ou 100 mg, dans chaque cas par rapport à la base libre de Tapentadol ; et dans laquelle la forme posologique a un poids total dans la plage de 150 à 750 mg.

6. Forme posologique pharmaceutique selon l'une quelconque des revendications 1 à 4, dans laquelle la dose équivalente en poids de Tapentadol contenue dans la forme posologique pharmaceutique est de 150 mg, 200 mg ou 250 mg, dans chaque cas par rapport à la base libre de Tapentadol ; et dans laquelle la forme posologique a un poids total dans la plage de 300 à 1 200 mg.

7. Forme posologique pharmaceutique selon l'une quelconque des revendications précédentes, dans laquelle la matrice à libération prolongée comprend ou est constituée sensiblement d'un matériau de matrice à libération prolongée choisi dans le groupe constitué de
(i) hydroxypropylmethylcellulose (HPMC) ;
(ii) hydroxypropylcellulose (HPC) ;
(iii) hydroxyéthylcellulose (HEC) ;
(iv) cellulose microcristalline (MCC) ;
(v) éthylcellulose (EC) ;
(vi) acétate de polyvinyle (PVAc) ;
(vii) polyvinylpyrrolidone (PVP) ;
(viii) copolymère polyvinylpyrrolidone-acétate de vinyle (PVP/PVAc) ;
(ix) poly(acrylate d'éthyle-co-méthacrylate de méthyle-co-chlorure de méthacrylate de triméthylammonioéthyle) ;
(x) poly(méthacrylate de butyle-co-(2-diméthylaminoéthyl) méthacrylate-co-méthyl méthacrylate) ;
(xi) poly(méthacrylate de méthyle-co-acide méthacrylique) ;
(xii) poly(acrylate d'éthyle-co-acide méthacrylique) ;
(xiii) poly(acrylate de méthyle-co-méthacrylate de méthyle-co-acide méthacrylique) ;
(xiv) poly(acrylate d'éthyle-co-méthacrylate de méthyle) ;
(xv) poly (oxyde d'éthylène) (PEO) ;
(xvi) polyéthylène glycol (PEG) ;
(xvii) alcool gras à longue chaîne ayant de 8 à 50 atomes de carbone, de préférence de 12 à 40 atomes de carbone, qui peut être saturé ou insaturé et linéaire ou ramifié ;
(xviii) alcool cétostéarylique ;
(xix) alcool stéarylique ;
(xx) alcool cétylique ;
(xxi) hydrocarbure choisi dans le groupe constitué des acides gras à longue chaîne ayant de 8 à 50 atomes de carbone, de préférence de 12 à 40 atomes de carbone, qui peuvent être saturés ou insaturés et linéaires ou ramifiés ; des esters de glycéryle de tels acides gras à longue chaîne, huiles minérales, huiles végétales et cires ;
(xxii) gomme xanthane ;
(xxiii) alginate de sodium ;
(xxiv) gomme de guar ;
(xxv) gomme de caroube ; et
tout mélange de ce qui précède.

8. Forme posologique pharmaceutique selon la revendication 7, dans laquelle la teneur en matériau de matrice à libération prolongée est de préférence dans la plage de 15±10 % en poids, ou 20±10 % en poids, ou 25±10 % en poids, ou 30±10 % en poids, ou 35±10 % en poids, ou 40±10 % en poids, ou 45±10 % en poids, ou 50±10 % en poids, ou 55±10 % en poids, ou 60±10 % en poids, ou 65±10 % en poids, ou 70±10 % en poids, ou 75±10 % en poids, ou 80±10 % en poids, dans chaque cas par rapport au poids total de la forme posologique.

9. Forme posologique pharmaceutique selon l'une quelconque des revendications précédentes pour une utilisation dans le traitement de la douleur.

10. Forme posologique pharmaceutique pour une utilisation selon la revendication 9, dans laquelle la forme posologique est administrée par voie orale.

11. Forme posologique pharmaceutique pour une utilisation selon la revendication 9 ou 10, dans laquelle la forme posologique est administrée deux fois par jour.

12. Forme posologique pharmaceutique pour une utilisation selon l'une quelconque des revendications 9 à 11, qui, après administration orale, fournit des taux plasmatiques de Tapentadol procurant un soulagement de la douleur pendant une durée d'au moins 6 heures.

13. Forme posologique pharmaceutique pour une utilisation selon l'une quelconque des revendications 9 à 12, dans laquelle la douleur est une douleur chronique.

14. Procédé de préparation d'une forme posologique pharmaceutique selon l'une quelconque des revendications 1 à 13, dans lequel le procédé comprend les étapes suivantes :
(a) fourniture d'un mélange contenant sensiblement la totalité de la quantité de Tapentadol devant être contenue dans la forme posologique et au moins un matériau de matrice à libération prolongée, éventuellement avec un ou plusieurs excipients ;
(b) éventuellement granulation du mélange fourni à l'étape (a), obtenant ainsi un granulé ;
(c) éventuellement mélange du granulé obtenu à l'étape (b) avec un ou plusieurs excipients, obtenant ainsi un mélange de granulés ;
(d) compression du mélange fourni à l'étape (a) ou du granulé obtenu à l'étape (b) ou du mélange de granulés obtenu à l'étape (c) en comprimés ;
(e) éventuellement, revêtement par film des comprimés compressés à l'étape (d).

15. Procédé selon la revendication 14, dans lequel la compression à l'étape (d) se produit avec une force de compression non supérieure à 20 kN, particulièrement de préférence non supérieure à 15 kN, encore plus de préférence non supérieure à 10 kN, encore plus de préférence non supérieure à 9,5 kN, encore plus de préférence non supérieure à 9,0 kN, encore plus de préférence non supérieure à 8,75 kN et en particulier non supérieure 8,5 kN.
